# EUROPEAN PATENT APPLICATION

(11) **EP 2 363 505 A2**
(43) Date of publication of application: **07.09.2011**
(21) Application number: 10191595.7
(22) Date of filing: 02.05.2008
(51) Int. Cl.: C12Q 1/68, C07H 21/04

(54) **Diagnosis of melanoma by nucleic acid analysis**

(30) Priority: 01.11.2007 US 984684 P; 03.01.2008 US 18850 P; 04.05.2007 US 927802 P; 07.05.2007 US 928248 P; 31.10.2007 US 984326 P
(62) Divisional of application: 08747580.2
(71) Applicant: Dermtech International, La Jolla, CA 92037-0068 (US)
(72) Inventor: Chang, Sherman H., San Diego, CA 92131 (US)
(74) Representative: Hollywood, Jane Constance

(57) **Abstract**

The present invention provides methods for diagnosing melanoma in a subject by analyzing nucleic acid molecules obtained from the subject. The present invention also provides methods for distinguishing melanoma from dysplastic nevi and/or normal pigmented skin. The methods include analyzing expression or mutations in epidermal samples, of one or more skin markers. The methods can include the use of a microarray to analyze gene or protein profiles from a sample.

## Description

### BACKGROUND OF THE INVENTION

### FIELD OF THE INVENTION

The invention relates generally to methods of characterizing pigmented skin lesions suspected of being melanomas using primarily non-invasive skin sampling.

### BACKGROUND INFORMATION

Melanoma is a serious form of skin cancer in humans. It arises from the pigment cells (melanocytes), usually in the skin. The incidence of melanoma is increasing at the fastest rate of all cancers in the United States with a lifetime risk of 1 in 68. Althought melanoma accounts for only 4% of all dermatologic cancers, it is responsible for 80% of all deaths from skin cancers. It has long been realized that recognition and diagnosis of melanoma, when it is early stage disease, is key to its cure.

Given this, it is imperative that research be carried out not only on therapeutics for melanoma, but also on all aspects of melanoma including prevention and detection. Most of these deaths from melanoma could have been prevented if the melanomas, initially located on the skin, could have been detected in their early stages. The ability to cure melanoma in its earliest skin stage, *in situ,* is virtually 100% if the melanoma is adequately surgically excised. If the melanoma is caught in a later stage, where it has invaded to a depth of 4 mm or more, the ten-year survival rate is less than 50%. If the melanoma is not detected until it has spread to distant parts of the body (Stage IV), the prognosis is dismal, with only 7-9% of patients surviving 5 years, with the median survival time being 8-9 months. The long-term "cure" rate for Stage IV melanoma is only 1-2 %.

To advance early detection of melanoma, several things must be improved. People need to be better educated with regards to the risks of melanoma and how to prevent and detect it on their own skin. Also physicians need to be more alert to the possibility of melanoma and be better trained in detection. But even if these two areas are improved, the diagnosis of melanoma on the skin is still difficult. Studies have shown that even expert clinicians working in pigmented lesion clinics where melanoma is their specialty are only able to determine whether a suspicious pigmented lesion is melanoma or not with 60-80% sensitivity. This leads to the need for surgical biopsy of large numbers of pigmented lesions for every melanoma that is detected, and, doubtless, to the missing of some melanomas in their early stages.

In current practice melanoma is diagnosed by biopsy and histopathological examination; approximately 20 to 30 biopsies must be performed to find one melanoma and even then some melanomas are missed in the earliest stage. The limitations of visual detection are apparent to dermatologists who are constantly searching for ways to better determine whether suspicious lesions are melanoma or not without having to cut them out first. To this end, epiluminescence microscopy (ELM) has come into use. This is a method whereby lesions are looked at using a device that simultaneously magnifies the lesion while reducing visual interference from refractive index differences at the skin-air interface. While ELM does give a different view, it is of limited improvement. Studies have shown that until one becomes fairly skilled in utilizing the instrument, sensitivity in detection of melanoma actually decreases. Even very skilled users of ELM improve their ability to detect melanomas only by 5-10%. This still leads to an unacceptable sensitivity in detection and the need to biopsy large numbers of benign lesions to detect a few melanomas. And again, some melanomas will be missed completely in their early stages.

Clearly there is a need for further development of technology that will enable physicians to determine the nature and extent of suspicious lesions of the skin. Such technology would ideally directly assay the physiology of the suspect lesion to enable a sensitive diagnosis.

### SUMMARY OF THE INVENTION

The present invention is based, in part, on the discovery that analysis of nucleic acid molecules or of protein expression products of nucleic acid molecules from specific genes can be used to characterize skin lesions in a subject. The method provides valuable genetic information based on DNA, messenger RNA, or protein expression products obtained therefrom, for example.

In one embodiment, the method involves use of a non-invasive approach for recovering nucleic acids such as DNA or messenger RNA or proteins from the surface of skin via a tape stripping procedure that permits a direct quantitative and qualitative assessment of biomarkers. Although tape-harvested nucleic acid and protein expression products are shown to be comparable in quality and utility to recovering such molecules by biopsy, the non-invasive method provides information regarding cells of the outermost layers of the skin that may not be obtained using biopsy samples. Finally, the non-invasive method is far less traumatic than a biopsy.

Thus, the non-invasive method is used to capture cells on pigmented skin lesions that are suspected of being early melanomas. Nucleic acid molecules obtained from skin cells captured by the non-invasive method are analyzed in order to diagnose the nature of the lesion (*e.g*., malignant melanoma). In one embodiment, a nucleic acid molecule is amplified prior to analysis. Secondary outcomes could include tests for diagnosis and prognosis of a variety of pigmented skin lesions and even to predict a therapeutic regimen. In another embodiment, the skin cells are lysed to extract one or more proteins, which are then quantitated to diagnose the nature of the lesion. It should be understood that the methods of the invention are not limited to non-invasive techniques for obtaining skin samples. For example, but not by limitation, one of skill in the art would know other techniques for obtaining a skin sample such as scraping of the skin, biopsy, suction, blowing and other techniques. As described herein, non-invasive tape stripping is an illustrative example for obtaining a skin sample.

In another embodiment, the methods involve detection of one or more mutations in the nucleic acid sequence of the nucleic acid molecule obtained from the skin. Such mutations may be a substitution, a deletion, and/or an insertion of the nucleic acid sequence that results in a diseased state in the subject from which the skin sample is obtained.

In one embodiment, the nucleic acid molecule analyzed is listed in Tables 1-6. For example, in one embodiment, the gene analyzed is any one or more of human mRNA for tyrosinase-related protein 1, LIM homeobox 2, melan-A, spermidine/spermine N1-acetyltransferase, NDRG family member 2, carbohydrate (N-acetylglucosamine-6-O) sulfotransferase 2, syndecan binding protein (syntenin), crystallin, alpha B, endothelin receptor type B, tetratricopeptide repeat domain 3, WAP four-disulfide core domain 3, cytochrome P450 (family 1, subfamily B, polypeptide 1), p8 protein (candidate of metastasis 1), interferon gamma-inducible protein 16, dopachrome tautomerase (dopachrome delta-isomerase, tyrosine-related protein 2), solute carrier family 39 (zinc transporter), member 6, heat shock 70kDa protein 2, vesicle-associated membrane protein 2 (synaptobrevin 2), Similar to AI661453 protein, and transient receptor potential cation channel (subfamily M, member 1), tyrosinase (oculocutaneous albinism IA), endothelin receptor type B, tetratricopeptide repeat domain 3, syndecan binding protein (syntenin), v-kit Hardy-Zuckerman 4 feline sarcoma viral oncogene homolog, OTU domain, ubiquitin aldehyde binding 1, protein phosphatase 1 regulatory (inhibitor) subunit 12A, calponin 2, dishevelled dsh homolog 3 (Drosophila), tribbles homolog 2 (Drosophila), mucolipin 3, actinin alpha 4, ribosomal protein S 15, CDC37 cell division cycle 37 homolog (S. cerevisiae), jumonji domain containing 3, v-maf musculoaponeurotic fibrosarcoma oncogene homolog B (avian), c-Maf-inducing protein, myosin heavy polypeptide 14, Hypothetical protein MGC40222, or combinations thereof. In one embodiment, the nucleic acid molecule is from one or more genes listed in Table 6.

Accordingly, provided herein is a method for characterizing and/or diagnosing melanoma in a subject, including obtaining a nucleic acid molecule or protein by biopsy of a skin lesion on the subject, and analyzing the nucleic acid molecule to distinguish melanoma from dysplastic nevi and/or normal pigmented skin in the subject. In this method, at least one nucleic acid molecule whose expression is informative of melanoma is detected in the epidermal sample. In one example, expression of one or more of the genes listed in Tables 1-6, or a combination thereof, is detected in the epidermal sample to characterize the melanoma. In one embodiment, the nucleic acid molecule is from one or more genes listed in any of Tables 1-6, or any combination thereof. In another embodiment, the gene is any one or more of human mRNA for tyrosinase-related protein 1, LIM homeobox 2, melan-A, spermidine/spermine N1-acetyltransferase, NDRG family member 2, carbohydrate (N-acetylglucosamine-6-O) sulfotransferase 2, syndecan binding protein (syntenin), crystallin, alpha B, endothelin receptor type B, tetratricopeptide repeat domain 3, WAP four-disulfide core domain 3, cytochrome P450 (family 1, subfamily B, polypeptide 1), p8 protein (candidate of metastasis 1), interferon gamma-inducible protein 16, dopachrome tautomerase (dopachrome delta-isomerase, tyrosine-related protein 2), solute carrier family 39 (zinc transporter), member 6, heat shock 70kDa protein 2, vesicle-associated membrane protein 2 (synaptobrevin 2), Similar to AI661453 protein, and transient receptor potential cation channel (subfamily M, member 1), tyrosinase (oculocutaneous albinism IA), endothelin receptor type B, tetratricopeptide repeat domain 3, syndecan binding protein (syntenin), v-kit Hardy-Zuckerman 4 feline sarcoma viral oncogene homolog, OTU domain, ubiquitin aldehyde binding 1, protein phosphatase 1 regulatory (inhibitor) subunit 12A, calponin 2, dishevelled dsh homolog 3 (Drosophila), tribbles homolog 2 (Drosophila), mucolipin 3, actinin alpha 4, ribosomal protein S 15, CDC37 cell division cycle 37 homolog (S. cerevisiaa), jumonji domain containing 3, v-maf musculoaponeurotic fibrosarcoma oncogene homolog B (avian), c-Maf-inducing protein, myosin heavy polypeptide 14, Hypothetical protein MGC40222, or combinations thereof. In one embodiment, the nucleic acid molecule is from one or more genes listed in Table 6.

The non-invasive methods of the invention involve applying an adhesive tape to a target area of skin in a manner sufficient to isolate a sample adhering to the adhesive tape, wherein the sample includes nucleic acid molecules or proteins. Typically, at least one nucleic acid molecule or protein whose expression is informative of melanoma is detected in the sample. The method of characterizing skin using tape stripping has a number of applications, such as the following: (i) disease classification/subclassification; (ii) monitoring disease severity and progression; (iii) monitoring treatment efficacy; and (iv) prediction of a particular treatment regimen. All of these applications, which themselves represent embodiments disclosed herein, preferably use non-invasive sampling to recover information that is otherwise difficult or impractical to recover (*e.g.*, through the use of biopsies). The information may be contained in the DNA, protein, or RNA of skin cells close to the surface of the skin. In one example, expression of one or more of the genes listed in Tables 1-6, or a combination thereof, is detected in the sample to characterize the sample. This exemplary method is particularly useful for distinguishing melanoma from dysplastic nevi and/or normal pigmented skin. In one embodiment, expression of one or more of the genes listed in Table 6 is detected in the sample to characterize the sample.

As such, also provided herein is a method for distinguishing melanoma from dysplastic nevi and/or normal pigmented skin in a subject, including applying an adhesive tape to a target area of skin in a manner sufficient to isolate a sample adhering to the adhesive tape, wherein the sample includes nucleic acid molecules. At least one nucleic acid molecule whose expression is informative of melanoma is detected in the sample. In one example, expression of one or more of the genes listed in Tables 1-6, or a combination thereof, is detected in the sample to characterize the melanoma. In one embodiment, expression of one or more of the genes listed in Table 6 is detected in the sample to characterize the melanoma.

Other embodiments are based in part on the discovery that for tape stripping of the skin, non-polar, pliable, adhesive tapes, especially pliable tapes with rubber adhesive, are more effective than other types of adhesive tapes. Using pliable tapes with rubber adhesives, as few as 10 or less tape strippings and in certain examples as few as 4 or even 1 tape stripping can be used to isolate and/or detect nucleic acid molecules from the epidermal layer of the skin.

In another embodiment, the methods of the invention provide for characterization of a skin lesion *in situ,* including application of a detectably labeled probe directly to a skin lesion for visual analysis. At least one nucleic acid molecule whose expression is informative of melanoma or dysplastic nevi or normal skin is detected on the skin lesion or surrounding margin or tissue using a specific probe. In one example, expression of one or more of the genes listed in Tables 1-6, or a combination thereof, is detected on the skin lesion or surrounding margin or tissue to characterize the melanoma. In one embodiment, expression of one or more of the genes listed in Table 6 is detected in the sample to characterize the melanoma.

Also provided herein is a method for diagnosing a disease state by establishing a gene expression pattern of a target area suspected of being melanoma on the skin of a subject and comparing the subject's gene expression profile to a reference gene expression profile obtained from a corresponding normal skin sample. In one embodiment, the target area of the skin simultaneously expresses a plurality of genes at the protein level that are markers for melanoma. In another embodiment, the genes are listed in Tables 1-6. In another embodiment, the genes are listed in Table 6.

In one embodiment, the method of diagnosing a disease state involves detection of one or more mutations in the nucleic acid sequence of the gene. Such mutations may be a substitution, a deletion, and/or an insertion of the nucleic acid sequence that results in a diseased state in the subject from which the skin sample is obtained. In one embodiment, the genes are listed in Tables 1-6. In another embodiment, the genes are listed in Table 6.

In another aspect, the invention provides kits for characterizing a skin lesion in a subject. In one embodiment, the kit includes a skin sample collection device, such as a biopsy needle or an adhesive tape for non-invasive tape stripping, and one or more probes or primers that selectively bind to one or more nucleic acid molecules in any of Tables 1-6, or to a nucleic acid or protein expression product of a nucleic acid molecule in any of Tables 1-6. For example, in one embodiment, the gene analyzed is any one or more of human mRNA for tyrosinase-related protein 1, LIM homeobox 2, melan-A, spermidine/spermine N1-acetyltransferase, NDRG family member 2, carbohydrate (N-acetylglucosamine-6-O) sulfotransferase 2, syndecan binding protein (syntenin), crystallin, alpha B, endothelin receptor type B, tetratricopeptide repeat domain 3, WAP four-disulfide core domain 3, cytochrome P450 (family 1, subfamily B, polypeptide 1), p8 protein (candidate of metastasis 1), interferon gamma-inducible protein 16, dopachrome tautomerase (dopachrome delta-isomerase, tyrosine-related protein 2), solute carrier family 39 (zinc transporter), member 6, heat shock 70kDa protein 2, vesicle-associated membrane protein 2 (synaptobrevin 2), Similar to AI661453 protein, and transient receptor potential cation channel (subfamily M, member 1), tyrosinase (oculocutaneous albinism IA), endothelin receptor type B, tetratricopeptide repeat domain 3, syndecan binding protein (syntenin), v-kit Hardy-Zuckerman 4 feline sarcoma viral oncogene homolog, OTU domain, ubiquitin aldehyde binding 1, protein phosphatase 1 regulatory (inhibitor) subunit 12A, calponin 2, dishevelled dsh homolog 3 (Drosophila), tribbles homolog 2 (Drosophila), mucolipin 3, actinin alpha 4, ribosomal protein S15, CDC37 cell division cycle 37 homolog (S. cerevisiae), jumonji domain containing 3, v-maf musculoaponeurotic fibrosarcoma oncogene homolog B (avian), c-Maf-inducing protein, myosin heavy polypeptide 14, Hypothetical protein MGC40222, or combinations thereof. The kit may include one or more pairs of forward primers that selectively bind upstream of a gene on one strand and reverse primers that selectively bind upstream of a gene on a complementary strand. In another embodiment, the kit includes a microarray containing at least a fragment of a gene or a nucleic acid or protein product of a gene identified in any of Tables 1-6 or any combination thereof.

In another embodiment, the kit for characterizing a skin lesion in a subject includes an applicator and one or more probes or primers that selectively bind to one or more nucleic acid molecules in any of Tables 1-6, or to a nucleic acid or protein expression product of a nucleic acid molecule in any of Tables 1-6, In one embodiment, the probes are detectably labeled for visual identification of expression of RNA.

### BRIEF DESCRIPTION OF THE DRAWINGS

Figures 1A and 1B are graphical diagrams showing data from the EDR, PTP, and PTN as a function of sample size, assuming a threshold for declaring the significance of a probe/gene expression difference between nevi and primary melanoma of p < 0.05.

Figures 2A and 2B are graphical diagrams showing data from a sample size analysis that considered the contrast results for nevi vs. primary melanoma in the context of an analysis of variance (ANOVA) comparing normal skin, nevi, and primary melanoma

Figures 3A and 3B are graphical diagrams showing data from an analysis focusing exclusively on the posterior true probability (PTP) for different assumed significance levels.

Figures 4A to 4D are pictorial and graphical diagrams showing the development of a gene classifier for distinguishing melanoma from atypical nevi and normal pigmented skin.

Figures 5A and 5B are graphical diagrams showing data from prediction analysis of the developed classifiers for distinguishing melanoma from atypical nevi and normal pigmented skin.

Figures 6A to 6E are graphical diagrams showing data from prediction analysis of the developed classifiers for distinguishing melanoma from atypical nevi and normal pigmented skin.

### DETAILED DESCRIPTION OF THE INVENTION

The present invention is based, in part, on the discovery that analysis of nucleic acid molecules or of protein expression products of nucleic acid molecules from specific genes can be used to characterize skin lesions in a subject. Accordingly, the present invention provides methods and kits useful for detecting cancer, especially melanoma by determining the expression profiles of one or more specific genes of interest.

There are two main motivations for conducting genome wide expression profiling studies in melanoma. First, melanoma is one of the best characterized carcinogenesis models for gradual progression of benign lesions to cancer: normal pigmented cells to nevi to atypical nevi to primary melanoma *in situ* to invasive primary melanoma to aggressive metastatic melanoma. This progression is known to correlate with distinctive chromosomal changes, and is thought to be mediated by stepwise progressive changes in gene expression, suggesting that expression profiling may identify genes responsible for tumorigenesis in melanoma. Indeed, candidate tumor genes have been identified with microarray analyses of melanoma cell lines. The second reason is that molecular characterization of tumors may allow a better staging classification of tumors and prognosis prediction. While histological characteristics such as the thickness and ulceration of tumors have some value as predictors of prognosis, there is lack of informative markers that help determine which patients will do well and which patients will have progressive disease and metastasis. Molecular markers identified in microarray experiments of tumors are already being introduced into clinical practice in the management of breast cancer. Gene expression profiling experiments in melanoma and melanoma cell lines suggest that the classification of melanoma can be improved, but studies are lacking with sufficient power to define molecular criteria for diagnosis or identify prognostic markers; the establishments of such markers would represent a major advance in melanoma care. A major reason for the lack of powerful microarray studies in melanoma is that, unlike most solid tumors, it is necessary to paraffin embed and section the whole lesion for histology, leaving no sample for RNA isolation. Although this situation is now changing, the ability to avoid biopsy until a definitive diagnosis is made would be powerful for subjects that would not normally be eligible for one or more biopsies.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

The term "subject" as used herein refers to any individual or patient to which the subject methods are performed. Generally the subject is human, although as will be appreciated by those in the art, the subject may be an animal. Thus other animals, including mammals such as rodents (including mice, rats, hamsters and guinea pigs), cats, dogs, rabbits, farm animals including cows, horses, goats, sheep, pigs, etc., and primates (including monkeys, chimpanzees, orangutans and gorillas) are included within the definition of subject.

As used herein, the terms "sample" and "biological sample" refer to any sample suitable for the methods provided by the present invention. A sample of cells can be any sample, including, for example, a skin sample obtained by non-invasive tape stripping or biopsy of a subject, or a sample of the subject's bodily fluid. Thus, in one embodiment, the biological sample of the present invention is a tissue sample, *e.g*., a biopsy specimen such as samples from needle biopsy. In one embodiment; the term "sample" refers to any preparation derived from skin of a subject. For example, a sample of cells obtained using the non-invasive method described herein can be used to isolate nucleic acid molecules or proteins for the methods of the present invention. Samples for the present invention typically are taken from a skin lesion, which is suspected of being the result of a disease or a pathological or physiological state, such as psoriasis or dermatitis, or the surrounding margin or tissue. As used herein, "surrounding margin" or "surrounding tissue" refers to tissue of the subject that is adjacent to the skin lesion, but otherwise appears to be normal or free from lesion.

As used herein "corresponding normal cells" or "corresponding normal sample" refers to cells or a sample from a subject that is from the same organ and of the same type as the cells being examined. In one aspect, the corresponding normal cells comprise a sample of cells obtained from a healthy individual that does not have a skin lesion or skin cancer. Such corresponding normal cells can, but need not be, from an individual that is age-matched and/or of the same sex as the individual providing the cells being examined. Thus, the term "normal sample" or "control sample" refers to any sample taken from a subject of similar species that is considered healthy or otherwise not suffering from the particular disease, pathological or physiological state, or from the same subject in an area free from skin lesions. As such, a normal/standard level of RNA denotes the level of RNA present in a sample from the normal sample. A normal level of RNA can be established by combining skin samples or cell extracts taken from normal healthy subjects and determining the level of one or more RNAs present. In addition, a normal level of RNA also can be determined as an average value taken from a population of subjects that is considered to be healthy, or is at least free of a particular disease, pathological or physiological state. Accordingly, levels of RNA in subject, control, and disease samples can be compared with the standard values. Deviation between standard and subject values establishes the parameters for diagnosing or characterizing disease.

The term "skin" refers to the outer protective covering of the body, consisting of the epidermis (including the stratum corneum) and the underlying dermis, and is understood to include sweat and sebaceous glands, as well as hair follicle structures. Throughout the present application, the adjective "cutaneous" can be used, and should be understood to refer generally to attributes of the skin, as appropriate to the context in which they are used. The epidermis of the human skin comprises several distinct layers of skin tissue. The deepest layer is the stratum basalis layer, which consists of columnar cells. The overlying layer is the stratum spinosum, which is composed of polyhedral cells. Cells pushed up from the stratum spinosum are flattened and synthesize keratohyalin granules to form the stratum granulosum layer. As these cells move outward, they lose their nuclei, and the keratohyalin granules fuse and mingle with tonofibrils. This forms a clear layer called the stratum lucidum. The cells of the stratum lucidum are closely packed. As the cells move up from the stratum lucidum, they become compressed into many layers of opaque squamae. These cells are all flattened remnants of cells that have become completely filled with keratin and have lost all other internal structure, including nuclei. These squamae constitute the outer layer of the epidermis, the stratum corneum. At the bottom of the stratum corneum, the cells are closely compacted and adhere to each other strongly, but higher in the stratum they become loosely packed, and eventually flake away at the surface.

As used herein, the term "skin lesion" refers to a change in the color or texture in an area of skin. As such, "skin lesions suspected of being melanoma" are skin lesions with characteristics of malignant melanoma, which are well known to those of skill in the art, such as dermatologists and oncologist. Such lesions are sometimes raised and can have a color that is different from the color of normal skin of an individual (*e.g*., brown, black, red, or blue). Lesions suspected of being melanoma sometimes include a mixture of colors, are often asymmetrical, can change in appearance over time, and may bleed. A skin lesion suspected of being melanoma may be a mole or nevus. Melanoma lesions are usually, but not always, larger than 6 mm in diameter. Melanoma includes superficial spreading melanoma, nodular melanoma, acral lentiginous melanoma, and lentigo-maligna melanoma. Melanoma can occur on skin that has been overexposed to the sun. Therefore, in one embodiment the skin sample is taken from an area of skin that has been overexposed to the sun.

The term "dysplastic nevus" refers to an atypical mole or a mole whose appearance is different from that of common moles. Dysplastic nevi are generally larger than ordinary moles and have irregular and indistinct borders. Their color frequently is not uniform and ranges from pink to dark brown; they usually are flat, but parts may be raised above the skin surface. Dysplastic naevus can be found anywhere, but are most common on the trunk of a subject.

The term "cancer" as used herein, includes any malignant tumor including, but not limited to, carcinoma and sarcoma. Cancer arises from the uncontrolled and/or abnormal division of cells that then invade and destroy the surrounding tissues. As used herein, "proliferating" and "proliferation" refer to cells undergoing mitosis. As used herein, "metastasis" refers to the distant spread of a malignant tumor from its sight of origin. Cancer cells may metastasize through the bloodstream, through the lymphatic system, across body cavities, or any combination thereof. The term "cancerous cell" as provided herein, includes a cell afflicted by any one of the cancerous conditions provided herein. The term "carcinoma" refers to a malignant new growth made up of epithelial cells tending to infiltrate surrounding tissues, and to give rise to metastases. The term "melanoma" refers to a malignant tumor of melanocytes which are found predominantly in skin but also in bowel and the eye. "Melanocytes" refer to cells located in the bottom layer, the basal lamina, of the skin's epidermis and in the middle layer of the eye. Thus, "melanoma metastasis" refers to the spread of melanoma cells to regional lymph nodes and/or distant organs (*e.g*., liver, brain, breast, prostate, etc.).

As used herein, the term "gene" refers to a linear sequence of nucleotides along a segment of DNA that provides the coded instructions for synthesis of RNA, which, when translated into protein, leads to the expression of hereditary character. As such, the term "skin marker" or "biomarker" refers to a gene whose expression level is different between skin surface samples at the site of malignant melanoma and skin surface samples of normal skin or a lesion, which is benign, such as a benign nevus. Therefore, expression of a melanoma skin marker of the invention is related to, or indicative of, melanoma. Many statistical techniques are known in the art, which can be used to determine whether a statistically significant difference in expression is observed at a high (*e.g*., 90% or 95%) confidence level. As such, an increase or decrease in expression of these genes is related to and can characterize malignant melanoma. In one embodiment, there is at least a two-fold difference in levels between skin sample near the site of malignant melanoma and skin samples from normal skin.

As used herein, the term "nucleic acid molecule" means DNA, RNA, single-stranded, double-stranded or triple stranded and any chemical modifications thereof. Virtually any modification of the nucleic acid is contemplated. A "nucleic acid molecle" can be of almost any length, from 10, 20, 30, 40, 50, 60, 75, 100, 125, 150, 175, 200, 225, 250, 275, 300, 400, 500, 600, 700, 800, 900, 1000, 1500, 2000, 2500, 3000, 3500, 4000, 4500, 5000, 6000, 7000, 8000, 9000, 10,000, 15,000,20,000, 30,000, 40,000, 50,000, 75,000, 100,000,150,000,200,000,500,000, 1,000,000, 1,500,000,2.000,000,5,000,000 or even more bases in length, up to a full-length chromosomal DNA molecule. For methods that analyze expression of a gene, the nucleic acid isolated from a sample is typically RNA.

Micro-RNAs (miRNA) are small single stranded RNA molecules an average of 22 nucleotides long that are involved in regulating mRNA expression in diverse species including humans (reviewed in Bartel 2004). The first report of miRNA was that of the *lin-4* gene, discovered in the worm *C*. *elegans* (Lee, Feinbaum et al. 1993). Since then hundreds of miRNAs have been discovered in flies, plants and mammals. miRNAs regulate gene expression by binding to the 3'-untranslated regions of mRNA and catalyze either i) cleavage of the mRNA; or 2) repression of translation. The regulation of gene expression by miRNAs is central to many biological processes such as cell development, differentiation, communication, and apoptosis (Reinhart, Slack et al. 2000; Baehrecke 2003; Brennecke, Hipfner et al. 2003; Chen, Li et al. 2004). Recently it has been shown that miRNA are active during embryogenesis of the mouse epithelium and play a significant role in skin morphogenesis (Yi, O'Carroll et al. 2006).

Given the role of miRNA in gene expression it is clear that miRNAs will influence, if not completely specify the relative amounts of mRNA in particular cell types and thus determine a particular gene expression profile (*i.e.*, a population of specific mRNAs) in different cell types. In addition, it is likely that the particular distribution of specific miRNAs in a cell will also be distinctive in different cell types. Thus, determination of the miRNA profile of a tissue may be used as a tool for expression profiling of the actual mRNA population in that tissue. Accordingly, miRNA levels and/or detection of miRNA mutations are useful for the purposes of disease detection, diagnosis, prognosis, or treatment-related decisions (*i.e.,* indicate response either before or after a treatment regimen has commenced) or characterization of a particular disease in the subject.

As used herein, the term "protein" refers to at least two covalently attached amino acids, which includes proteins, polypeptides, oligopeptides and peptides. A protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and noreleucine are considered amino acids for the purposes of the invention. "Amino acid" also includes imino acid residues such as proline and hydroxyproline. The side chains may be in either the (R) or the (S) configuration.

A "probe" or "probe nucleic acid molecule" is a nucleic acid molecule that is at least partially single-stranded, and that is at least partially complementary, or at least partially substantially complementary, to a sequence of interest. A probe can be RNA, DNA, or a combination of both RNA and DNA. It is also within the scope of the present invention to have probe nucleic acid molecules comprising nucleic acids in which the backbone sugar is other that ribose or deoxyribose. Probe nucleic acids can also be peptide nucleic acids. A probe can comprise nucleolytic-activity resistant linkages or detectable labels, and can be operably linked to other moieties, for example a peptide.

A single-stranded nucleic acid molecule is "complementary" to another single-stranded nucleic acid molecule when it can base-pair (hybridize) with all or a portion of the other nucleic acid molecule to form a double helix (double-stranded nucleic acid molecule), based on the ability of guanine (G) to base pair with cytosine (C) and adenine (A) to base pair with thymine (T) or uridine (U). For example, the nucleotide sequence 5'-TATAC-3' is complementary to the nucleotide sequence 5'-GTATA-3'.

The term "antibody" as used in this invention is meant to include intact molecules of polyclonal or monoclonal antibodies, as well as fragments thereof, such as Fab and F(ab')₂, Fv and SCA fragments which are capable of binding an epitopic determinant. The term "specifically binds" or "specifically interacts," when used in reference to an antibody means that an interaction of the antibody and a particular epitope has a dissociation constant of at least about 1 x 10⁻⁶, generally at least about 1 x 10⁻⁷, usually at least about 1 x 10⁻⁸, and particularly at least about 1 x 10⁻⁹ or 1 x 10⁻¹⁰ or less.

As used herein "hybridization" refers to the process by which a nucleic acid strand joins with a complementary strand through base pairing. Hybridization reactions can be sensitive and selective so that a particular sequence of interest can be identified even in samples in which it is present at low concentrations. In an *in vitro* situation, suitably stringent conditions can be defined by, for example, the concentrations of salt or formamide in the prehybridization and hybridization solutions, or by the hybridization temperature, and are well known in the art. In particular, stringency can be increased by reducing the concentration of salt, increasing the concentration of formamide, or raising the hybridization temperature. For example, hybridization under high stringency conditions could occur in about 50% formamide at about 37°C to 42°C. Hybridization could occur under reduced stringency conditions in about 35% to 25% formamide at about 30°C to 35°C. In particular, hybridization could occur under high stringency conditions at 42°C in 50% formamide, 5X SSPE, 0.3% SDS, and 200 mg/ml sheared and denatured salmon sperm DNA. Hybridization could occur under reduced stringency conditions as described above, but in 35% formamide at a reduced temperature of 35°C, The temperature range corresponding to a particular level of stringency can be further narrowed by calculating the purine to pyrimidine ratio of the nucleic acid of interest and adjusting the temperature accordingly. Variations on the above ranges and conditions are well known in the art.

As used herein, the term "mutation" refers to a change in the genome with respect to the standard wild-type sequence. Mutations can be deletions, insertions, or rearrangements of nucleic acid sequences at a position in the genome, or they can be single base changes at a position in the genome, referred to as "point mutations." Mutations can be inherited, or they can occur in one or more cells during the lifespan of an individual.

As used herein, the term "kit" or "research kit" refers to a collection of products that are used to perform a biological research reaction, procedure, or synthesis, such as, for example, a detection, assay, separation, purification, etc., which are typically shipped together, usually within a common packaging, to an end user.

As used herein, the term "ameliorating" or "treating" means that the clinical signs and/or the symptoms associated with the cancer or melanoma are lessened as a result of the actions performed. The signs or symptoms to be monitored will be characteristic of a particular cancer or melanoma and will be well known to the skilled clinician, as will the methods for monitoring the signs and conditions. Thus, a "treatment regimen" refers to any systematic plan or course for treating a disease or cancer in a subject.

Samples from a tissue can be isolated by any number of means well known in the art. Invasive methods for isolating a sample include, but are not limited to the use of needles or scalpels, for example during biopsies of various tissues. Non-invasive methods for isolating a sample include, but are not limited to tape-stripping and skin scraping.

Accordingly, in one embodiment, the present invention employs a non-invasive tape stripping technology to obtain samples of suspicious lesions. As such, DNA microarray assays are used to create a non-invasive diagnostic for melanoma. Tape-stripping removes superficial cells from the surface of the skin as well as adnexal cells. Small amounts of nucleic acid molecules isolated from tape-stripped cells can be amplified and used for microarray analyses and quantitative PCR. In addition, proteins obtained from the lysed cells may be quantitated for diagnosis of disease. Consequently, tape-stripping is a non-invasive diagnostic method, which does not interfere with subsequent histological analyses, thereby bypassing a major limitation to current expression profiling studies on melanoma, While tape stripping will primarily sample superficial cells from the epidermis, this method holds great promise in the diagnoses and prognosis prediction in pigmented lesions for the following reasons: First, in contrast to benign nevi, in many melanomas the pigmented cells migrate into the epidermis and/or adnexa. Consequently, this feature may help differentiate benign pigmented lesions from melanomas based on tape stripping. Second, there are changes in the dermis and epidermis adj acent to melanoma. The epidermal hyperplasia overlying melanoma seems to correlate with both angiogenesis and metastatic potential; these changes are expected to be sampled with the tape stripping method. Finally, some advanced melanomas do reach the surface of the skin and melanoma cancer cells would be sampled directly by the tape stripping. In addition tape stripping is useful in the care of patients with multiple pigmented lesions where it is unpractical to biopsy each and every lesion. Accordingly, the present invention demonstrates that stratum corneum RNA, harvested by tape stripping with Epidermal Genetic Information Retrieval (EGIR) (see U.S. Pat. No. 6,949,338, incorporated herein by reference), can be used to distinguish melanoma from dysplastic nevi in suspicious pigmented lesions.

As indicated, the tape stripping methods provided herein typically involve applying an adhesive tape to the skin of a subject and removing the adhesive tape from the skin of the subject one or more times. In certain examples, the adhesive tape is applied to the skin and removed from the skin about one to ten times. Alternatively, about ten adhesive tapes can be sequentially applied to the skin and removed from the skin. These adhesive tapes are then combined for further analysis. Accordingly, an adhesive tape can be applied to and removed from a target site 10, 9, 8, 7, 6, 5, 4, 3, 2, or 1 time, and/or 10, 9, 8, 7, 6, 5, 4, 3, 2, or 1 adhesive tape can be applied to and removed from the target site. In one illustrative example, the adhesive tape is applied to the skin between about one and eight times, in another example, between one and five times, and in another illustrative example the tape is applied and removed from the skin four times.

The rubber based adhesive can be, for example, a synthetic rubber-based adhesive. The rubber based adhesive in illustrative examples, has high peel, high shear, and high tack. For example, the rubber based adhesive can have a peak force tack that is at least 25%, 50%, or 100% greater than the peak force tack of an acrylic-based tape such as D-SQUAME™. D-SQUAME ™ has been found to have a peak force of 2 Newtons, wherein peak force of the rubber based adhesive used for methods provided herein, can be 4 Newtons or greater. Furthermore, the rubber based adhesive can have adhesion that is greater than 2 times, 5 times, or 10 times that of acrylic based tape. For example, D-SQUAME ™ has been found to have adhesion of 0.0006 Newton meters, whereas the rubber based tape provided herein can have an adhesion of about 0.01 Newton meters using a texture analyzer. Furthermore, in certain illustrative examples, the adhesive used in the methods provided herein has higher peel, shear and tack than other rubber adhesives, especially those used for medical application and Duct tape.

Virtually any size and/or shape of adhesive tape and target skin site size and shape can be used and analyzed, respectively, by the methods of the present invention. For example, adhesive tape can be fabricated into circular discs of diameter between 10 millimeters and 100 millimeters, for example between 15 and 25 millimeters in diameter. The adhesive tape can have a surface area of between about 50 mm² and 1000 mm², between about 100 mm² to 500 mm² or about 250 mm².

In antoher embodiment, the sample is obtained by means of an invasive procedure, such as biopsy. Biopsies may be taken instead of or after tape stripping and are subjected to standard histopathologic analysis. Analysis of biopsy samples taken simultaneously with tape stripping samples may then be correlated with the data generated from one or more of analysis of selected lesion RNA samples by DNA microarray, correlation of gene expression data with histopathology, and creation of a candidate expression classifier for diagnosis of melanoma.

As used herein, "biopsy" refers to the removal of cells or tissues for analysis. There are many different types of biopsy procedures known in the art. The most common types include: (1) incisional biopsy, in which only a sample of tissue is removed; (2) excisional biopsy, in which an entire lump or suspicious area is removed; and (3) needle biopsy, in which a sample of tissue or fluid is removed with a needle. When a wide needle is used, the procedure is called a core biopsy. When a thin needle is used, the procedure is called a fine-needle aspiration biopsy. Other types of biopsy procedures include, but are not limited to, shave biopsy, punch biopsy, curettage biopsy, and *in situ* biopsy. In another embodiment, the skin sample is obtained by scraping the skin with an instrument to remove one or more nucleic acid molecules from the skin.

The skin sample obtained using the tape stripping method includes, epidermal cells including cells comprising adnexal structures. In certain illustrative examples, the sample includes predominantly epidermal cells, or even exclusively epidermal cells. The epidermis consists predominantly of keratinocytes (> 90%), which differentiate from the basal layer, moving outward through various layers having decreasing levels of cellular organization, to become the cornified cells of the stratum corneum layer. Renewal of the epidermis occurs every 20-30 days in uninvolved skin. Other cell types present in the epidermis include melanocytes, Langerhans cells, and Merkel cells. As illustrated in the Examples herein, the tape stripping method of the present invention is particularly effective at isolating epidermal samples.

Nucleic acid molecules can also be isolated by lysing the cells and cellular material collected from the skin sample by any number of means well known to those skilled in the art. For example, a number of commercial products available for isolating polynucleotides, including but not limited to, RNeasy™ (Qiagen, Valencia, CA) and TriReagent™ (Molecular Research Center, Inc, Cincinnati, OH) can be used. The isolated polynucleotides can then be tested or assayed for particular nucleic acid sequences, including a polynucleotide encoding a cytokine. Methods of recovering a target nucleic acid molecule within a nucleic acid sample are well known in the art, and can include microarray analysis.

Nucleic acid molecules may be analyzed in any number of ways known in the art. For example, the presence of nucleic acid molecules can be detected by DNA-DNA or DNA-RNA hybridization or amplification using probes or fragments of the specific nucleic acid molecule. Nucleic acid amplification based assays involve the use of oligonucleotides or oligomers based on the nucleic acid sequences to detect transformants containing the specific DNA or RNA.

In one embodiment, analysis of the nucleic acid molecules includes genetic analysis is to determine the nucleotide sequence of a gene. Since a difference in length or sequence between DNA fragments isolated from a sample and those of known sequences are due to an insertion, deletion, or substitution of one or more nucleotides, the determination of nucleic acid sequences provides information concerning mutations which have absolute influence on the physiology of the disease state in the subject. These mutations may also include transposition or inversion and are difficult to detect by other techniques than direct sequencing. For example, it has recently been shown that the presence of the c-kit-activating mutation, L576P, is indicative of malignant melanomas (see Table 1). Accordingly, the methods of the present invention may be used to detect genetic mutations in one or more genes listed in Tables 1-6 for diagnosis and/or characterization of a skin lesion in a subject.

A variety of protocols for detecting and measuring the expression of nucleic acid molecules, using either polyclonal or monoclonal antibodies specific for the protein expression product are known in the art. Examples include enzyme-linked immunosorbent assay (ELISA), radioimmunoassay (RIA), and fluorescence activated cell sorting (FACS). These and other assays are described, among other places, in Hampton, R. et al. (1990; Serological Methods, a Laboratory Manual, APS Press, St Paul, Minn.) and Maddox, D. E. et al. (1983; J. Exp. Med. 158:1211-1216).

In another embodiment, antibodies that specifically bind the expression products of the nucleic acid molecules of the invention may be used to characterize the skin lesion of the subject. The antibodies may be used with or without modification, and may be labeled by joining them, either covalently or non-covalently, with a reporter molecule.

A wide variety of labels and conjugation techniques are known by those skilled in the art and may be used in various nucleic acid and amino acid assays. Means for producing labeled hybridization or PCR probes for detecting sequences related to the nucleic acid molecules of Tables 1-6 include oligolabeling, nick translation, end-labeling or PCR amplification using a labeled nucleotide. Alternatively, the nucleic acid molecules, or any fragments thereof, may be cloned into a vector for the production of an mRNA probe. Such vectors are known in the art, are commercially available, and may be used to synthesize RNA probes *in vitro* by addition of an appropriate RNA polymerase such as T7, T3, or SP6 and labeled nucleotides. These procedures may be conducted using a variety of commercially available kits (Pharmacia & Upjohn, (Kalamazoo, Mich.); Promega (Madison Wis.); and U.S. Biochemical Corp., Cleveland, Ohio). Suitable reporter molecules or labels, which may be used for ease of detection, include radionuclides, enzymes, fluorescent, chemiluminescent, or chromogenic agents as well as substrates, cofactors, inhibitors, magnetic particles, and the like.

PCR systems usually use two amplification primers and an additional amplicon-specific, fluorogenic hybridization probe that specifically binds to a site within the amplicon. The probe can include one or more fluorescence label moieties. For example, the probe can be labeled with two fluorescent dyes: 1) a 6-carboxy-fluorescein (FAM), located at the 5'-end, which serves as reporter, and 2) a 6-carboxy-tetramethyl-rhodamine (TAMRA), located at the 3'-end, which serves as a quencher. When amplification occurs, the 5'-3' exonuclease activity of the Taq DNA polymerase cleaves the reporter from the probe during the extension phase, thus releasing it from the quencher. The resulting increase in fluorescence emission of the reporter dye is monitored during the PCR process and represents the number of DNA fragments generated. *In situ* PCR may be utilized for the direct localization and visualization of target nucleic acid molecules and may be further useful in correlating expression with histopathological finding.

Means for producing specific hybridization probes for nucleic acid molecules of the invention include the cloning of the nucleic acid sequences into vectors for the production of mRNA probes. Such vectors are known in the art, commercially available, and may be used to synthesize RNA probes *in vitro* by means of the addition of the appropriate RNA polymerases and the appropriate labeled nucleotides. Hybridization probes may be labeled by a variety of reporter groups, for example, radionuclides such as 32P or 35S, or enzymatic labels, such as alkaline phosphatase coupled to the probe via avidin/biotin coupling systems, and the like.

In order to provide a basis for the diagnosis or characterization of disease associated with expression of the nucleic acid molecules of the invention, a normal or standard profile for expression is established. Standard hybridization may be quantified by comparing the values obtained from subjects of known skin characterization (*e.g.*, from subjects either having melanoma or having dysplastic nevi). Standard values obtained from such samples may be compared with values obtained from samples from subjects having skin lesions that are suspected of being melanoma. Deviation between standard and subject values is used to establish the presence of disease.

Accordingly, in one aspect of the invention, a non-invasive sampling method is provided for the characterization of skin lesion on the skin. In one embodiment, a sample set of pigmented skin lesions is created. Each sample consists of nucleic acid molecules recovered by tape stripping or biopsy sample of the superficial epidermis overlying the lesion. In addition to tape striping, a standard biopsy of the same lesion may also be performed, along with accompanying histology and diagnosis. Nucleic acid molecules recovered by tape stripping the superficial epidermis of normal skin will serve as a negative control.

In another aspect, the invention provides a method of distinguishing melanoma from dysplastic nevi and/or normal pigmented skin in a subject. In one embodiment, the method includes analyzing a nucleic acid molecule from one or more genes listed in any of Tables 1-6, or any combination thereof. A target area of the skin of a subject that suspected of being melanoma is assayed for expression of a large number of genes. Analyzing expression includes any qualitative or quantitative method for detecting expression of a gene, many of which are known in the art. The method can include analyzing expression of specific markers by measuring expression of the markers using a quantitative method, or by using a qualitative method. Non-limiting methods for analyzing polynucleotides and polypeptides are discussed below.

Methods of analyzing expression of a gene of the present invention can utilize a microarray, or other miniature high-throughput technology, for detecting expression of one or more gene products. Quantitative measurement of expression levels using such microarrays is also known in the art, and typically involves a modified version of a traditional method for measuring expression as described herein. For example, such quantitation can be performed by measuring a phosphor image of a radioactive-labeled probe binding to a spot of a microarray, using a phospohor imager and imaging software.

In a related aspect, the invention provides a method for diagnosing various disease states in a subject by identifying new diagnostic markers, specifically the classification and diagnosis of melanoma. By identifying gene sets that are unique to a given state, these differences in the genetic expression can be utilized for diagnostic purposes. In one embodiment, the nucleic acid molecule is RNA, including messenger RNA (mRNA) that is isolated from a sample from the subject. Up-regulated and down-regulated gene sets for a given disease state may be subsequently combined. The combination enables those of skill in the art to identify gene sets or panels that are unique to a given disease state. Such gene sets are of immense diagnostic value as they can be routinely used in assays that are simpler than microarray analysis (for example "real-time" quantitative PCR). Such gene sets also provide insights into pathogenesis and targets for the design of new drugs.

A reference database containing a number of reference projected profiles is also created from skin samples of subjects with known states, such as normal (*i.e*., non-melanoma) and various skin cancer disease states. The projected profile is then compared with the reference database containing the reference projected profiles. If the projected profile of the subject matches best with the profile of a particular disease state in the database, the subject is diagnosed as having such disease state. Various computer systems and software can be utilized for implementing the analytical methods of this invention and are apparent to one of skill in the art. Exemplary software programs include, but are not limited to, Cluster & TreeView (Stanford, URLs: rana.lbl.gov or microarray.org), GeneCluster (MIT/Whitshead Institute, URL: MPR/GeneCluster/GeneCluster.html), Array Explorer (SpotFire Inc, URL: spotfire.conVproducts/scicomp.asp#SAE) and GeneSpring (Silicon Genetics Inc, URL: sigenetics.com/Products/GeneSprmg/index.html) (for computer systems and software, see also U.S. Pat. No. 6,203,987, incorporated herein by reference).

In another aspect, the methods of the present invention involve *in situ* analysis of the skin lesion for characterization thereof. For *in situ* methods, nucleic acid molecules do not need to be isolated from the subject prior to analysis. In one embodiment, detectably labeled probes are contacted with a cell or tissue of a subject for visual detection of expressed RNA to characterize the skin lesion.

In another aspect, the methods of the present invention can also be useful for monitoring the progression of diseases and the effectiveness of treatments. For example, by comparing the projected profile prior to treatment with the profile after treatment. In one embodiment, the method characterizes a cancer as melanoma metastasis based on analysis of one or more nucleic acid molecules from Tables 1-6. It is known that in many cases, by the time a diagnosis of melanoma is established in a subject, metastasis has already occurred since melanomas contain multiple cell populations characterized by diverse growth rates, karyotypes, cell-surface properties, antigenicity, immunogenicity, invasion, metastasis, and sensitivity to cytotoxic drugs or biologic agents. Thus, the present invention may be used to characterize cancer of an organ as having metastasized from melanoma.

In a related aspect, the methods of the present invention can also be useful for determining an appropriate treatment regimen for a subject having a specific cancer or melanoma. Thus, the methods of the invention are useful for providing a means for practicing personalized medicine, wherein treatment is tailored to a subject based on the particular characteristics of the cancer in the subject. The method can be practiced, for example, by first determining whether the skin lesion is melanoma, as described above.

The sample of cells examined according to the present method can be obtained from the subject to be treated, or can be cells of an established cancer cell line of the same type as that of the subject. In one aspect, the established cell line can be one of a panel of such cell lines, wherein the panel can include different cell lines of the same type of disease and/or different cell lines of different diseases associated with expression of the genes of interest. Such a panel of cell lines can be useful, for example, to practice the present method when only a small number of cells can be obtained from the subject to be treated, thus providing a surrogate sample of the subject's cells, and also can be useful to include as control samples in practicing the present methods.

Once disease is established and a treatment protocol is initiated, the methods of the invention may be repeated on a regular basis to monitor the expression profiles of the genes of interest in the subject. The results obtained from successive assays may be used to show the efficacy of treatment over a period ranging from several days to months. Accordingly, another aspect of the invention is directed to methods for monitoring a therapeutic regimen for treating a subject having skin cancer. A comparison of the expression profile or mutations in the nucleic acid sequence of the nucleic acid molecule prior to and during therapy will be indicative of the efficacy of the therapy. Therefore, one skilled in the art will be able to recognize and adjust the therapeutic approach as needed.

The efficacy of a therapeutic regimen for treating a cancer over time can be identified by an absence of symptoms or clinical signs of the particular cancer in a subject at the time of onset of therapy. In subjects diagnosed as having the particular cancer, the efficacy of a method of the invention can be evaluated by measuring a lessening in the severity of the signs or symptoms in the subject or by the occurrence of a surrogate end-point for the disorder.

In addition, such methods may help identify an individual as having a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

When performed in a high throughput (or ultra-high throughput) format, the methods of the invention can be performed on a solid support (*e.g*., a microtiter plate, a silicon wafer, or a glass slide), wherein cell samples and/or genes of interest are positioned such that each is delineated from each other (*e.g*., in wells). Any number of samples or genes (*e.g.,* 96, 1024, 10,000, 100,000, or more) can be examined in parallel using such a method, depending on the particular support used. Where samples are positioned in an array (*i.e*., a defined pattern), each sample in the array can be defined by its position (*e.g*., using an x-y axis), thus providing an "address" for each sample. An advantage of using an addressable array format is that the method can be automated, in whole or in part, such that cell samples, reagents, genes of interest, and the like, can be dispensed to (or removed from) specified positions at desired times, and samples (or aliquots) can be monitored, for example, for expression products and/or mutations in the nucleic acid sequence of the nucleic acid molecules from any one of the genes listed in Tables 1-6.

Thus, the microarray can be used to monitor the expression level of large numbers of genes simultaneously (to produce a transcript image), and to identify genetic variants, mutations and polymorphisms. Polynucleotides used in the microarray may be oligonucleotides that are specific to a gene or genes of interest in which at least a fragment of the sequence is known or that are specific to one or more unidentified cDNAs which are common to a particular cell type, developmental or disease state. In order to produce oligonucleotides to a known sequence for a microarray, the gene of interest is examined using a computer algorithm which starts at the 5' or more preferably at the 3' end of the nucleotide sequence. The algorithm identifies oligomers of defined length that are unique to the gene, have a GC content within a range suitable for hybridization, and lack predicted secondary structure that may interfere with hybridization. In certain situations it may be appropriate to use pairs of oligonucleotides on a microarray. The "pairs" will be identical, except for one nucleotide which preferably is located in the center of the sequence. The second oligonucleotide in the pair (mismatched by one) serves as a control. The number of oligonucleotide pairs may range from two to one million. The oligomers are synthesized at designated areas on a substrate using a light-directed chemical process. The substrate may be paper, nylon or other type of membrane, filter, chip, glass slide or any other suitable solid support.

According to another aspect of the present invention, a kit is provided that is useful for detecting cancer in a cell or tissue, *e.g*., using the methods provided by the present invention for characterizing a skin lesion in a subject. In one embodiment, a kit of the invention includes a skin sample collection device and one or more probes or primers that selectively bind to one or more of the nucleic acid molecules in any of Tables 1-6. In another embodiment, the kit includes one or more applicators in addition to or instead of the skin sample collection device. Such applicators are useful for *in situ* analysis of gene expression on the skin of a subject. For example, an applicator may be used to apply detectably labeled probes for visual detection of expressed RNA to characterize the skin lesion.

In another embodiment, a kit of the invention includes a probe that binds to a portion of a nucleic acid molecule in any of Tables 1-6. In another embodiment, the kit further includes a microarray that contains at least a fragment of a gene or a nucleic acid molecule or a protein product of any one of the genes listed in Tables 1-6. In some embodiments, many reagents may be provided in a kit of the invention, only some of which should be used together in a particular reaction or procedure. For example, multiple primers may be provided, only two of which are needed for a particular application.

In another embodiment, the kit of the invention provides a compartmentalized carrier including a first container containing a pair of primers. The primers are typically a forward primer that selectively binds upstream of a gene on one strand, and a reverse primer that selectively binds upstream of a gene on a complementary strand. Optionally the kits of the present invention can further include an instruction insert, *e.g*., disclosing methods for sample collection using the sample collection device and/or exemplary gene expression profiles for comparison with the expression profile of the sample taken from the subject.

The following examples are provided to further illustrate the advantages and features of the present invention, but are not intended to limit the scope of the invention. While they are typical of those that might be used, other procedures, methodologies, or techniques known to those skilled in the art may alternatively be used.

### EXAMPLE 1

### RNA Quantitation and Profiling

The core hypothesis of this study is that the overlying epidermis of an early melanoma can be recovered with tape and that RNA contained within this sample is different than nearby epidermal RNA, *i.e.* that this RNA is diagnostic because of the underlying melanoma. Such a change in gene expression is documented for more advanced melanomas (McCarty et al., 2003) and is presumed to be true for early melanomas.

This study is divided into two separate phases, a sample collection and characterization phase (phase 1) and an RNA profiling phase (phase 2). In phase 1 the tape stripped specimens and biopsied sample collections were performed by the principal investigator or trained individuals delegated by the principal investigator to obtain the biopsy sample at various sites. All biopsies are subject to standard histopathologic analysis. The RNA profiling phase (Phase 2), includes, but is not limited to RNA purification and hybridization to DNA microarrays for gene expression profiling,

**Materials and reagents.** Adhesive tape was purchased from Adhesives Research (Glen Rock, PA) in bulk rolls. These rolls were custom fabricated into small circular discs, 17 millimeters in diameter, by Diagnostic Laminations Engineering (Oceanside, CA). Human spleen total RNA was purchased from Ambion (catalogue # 7970; Austin, TX). RNeasy RNA extraction kit was purchased from Qiagen (Valencia, CA). Reverse transcriptase, PCR primers and probes, and TaqMan Universal Master Mix, which included all buffers and enzymes necessary for the amplification and fluorescent detection of specific cDNAs, were purchased from Applied Biosystems (Foster City, CA). MELT total nucleic acid isolation system was purchased from Ambion (Austin, TX).

**RNA isolation.** RNA was extracted from tapes using either pressure cycling technology (PCT; Garrett, Tao et al. 2002; Schumacher, Manak et al. 2002) or MELT total nucleic acid system. Tapes were extracted in pairs by insertion into a PULSE™ tube (Pressure Biosciences, Gaithersburg, MD) with 1.2 mls of buffer RLT (supplied in the Qiagen RNeasy kit). PULSE™ tubes were inserted into the PCT-NEP2017 pressure cycler and the sample was extracted using the following parameters: room temperature; 5 pressure cycles of 35 Kpsi with pressure held for 20 seconds at the top and bottom of each cycle. After pressure extraction the buffer was removed and used to process the remaining tapes used to strip that site; the buffer was then processed according to the standard Qiagen RNeasy protocol for the collection of larger RNAs (>200 nucleotides) by application to a purification column to which large RNA molecules (*i.e.* mRNAs) bind, while the column flow-through is saved for microRNA purification. The column flow-through, which contains miRNA separated from mRNA, is processed according to the Qiagen mRNA purification procedure (on the world wide web at qiagen.com/literature/protocols/pdf/RY20.pdf) to purify the microRNA. RNA from the 2 sites stripped on each subject was pooled to create a single sample from each subject.

**RNA isolation using MELT total nucleic acid protocol.** Tapes were extracted in a 2 ml eppendorf tube with 192 ml MELT buffer plus 8 ml of MELT cocktail and vortexed for 10 minutes at room temperature. The MELT lysates were transferred to the dispensed binding bead master mix after spinning down for 3 minutes at >10,000 xg and washed with 300 ml of Wash Solution 1 and 2. RNA were eluted in 100 ml of elution solution.

**Quantitation of RNA.** Experimental data is reported as the number of PCR cycles required to achieve a threshold fluorescence for a specific cDNA and is described as the "Cₜ" value (Gibson, Heid et al. 1996; Heid, Stevens et al. 1996; AppliedBiosystenas 2001). Quantitation of total RNA mass was performed as previously described (Wong, Tran et al. 2004). Briefly, RNA mass recovered from tapes is determined by using quantitative RT-PCR with reference to a standard curve (Cₜ, actin vs. log [RNA]; AppliedBiosystems 2001) created from commercially purchased human spleen total RNA. The average of 6 replicate Cₜ, actin values was used to calculate the concentration of RNA in a sample with reference to the standard curve.

**RNA amplification and array hybridization.** RNA was isolated by the Multi-Enzymatic Liquefaction of Tissue method (Ambion, Austin, TX) and amplified using the WT-Ovation pico amplification system (NuGen, San Carlos, CA). The amplified RNA was hybridized to Affymetrix U133 plus 2.0 microarray and data were processed and analyzed using R from Bioconductor.

**Sample size.** Sample size calculations are presented in Example 2. This analysis predicts that in order to find 25 - 40 genes with high predictive value (p < 0.001) for discriminating benign nevi from melanoma then approximately 30 melanomas and 30 non-melanoma lesions are needed.

**Preprocessing GeneChip Data.** The image files from scanning the Affymetrix GeneChips with the Affymetrix series 3000 scanner will be converted using GCOS software (Affymetrix) to "CEL" format files. Normalization of CEL files will be carried out using software from the Bioconductor suite (on the world wide web at bioconductor.org). In particular, a robust multiarray analysis with adjustments for optical noise and binding affinities of oligonucleotide probes (Wu et al., 2006; and Wu et al., 2004) as implemented by the function "just.germa" in the "germa" package will be used to normalize the GeneChip Data.

**Statistical Approach for Microarray Data Analysis.** Two generic statistical problems are addressed in this proposal: (i) identifying genes that are differentially expressed in different classes of lesions (*i.e*. melanoma versus non-melanoma lesions) and (ii) forming (and evaluating) rules for classification of melanoma and non-melanoma lesions into groups based on gene expression data.

The most important grouping divides melanoma from non-melanoma on the basis of biopsy results. The methods that will be used to address the problems identified above are now standard in the statistical evaluation of micro array data (for reviews see Smyth et al., 2003; and Lee, 2004)). These methods have been applied by others to data from Affymetrix arrays to study gene expression in prostate cancer (Stuart et al., 2004), to characterize changes in gene expression subsequent to HIV infection (Mitchell et al., 2003), and to develop a high throughput genotyping platform (Wolyn et al_{.}, 2004; and Borevitz et al., 2003). For identifying differentially expressed genes, permutation based estimates of false discovery rates (reviewed in Efron et al., 2002) are preferred. Scripts for the R quantitative programming environment were developed to implement these methods in our previous work, but will likely use or adapt the "siggenes" package from the Bioconductor suite in this project. The development of classification rules will rely on resampling methods (k-fold cross-validation, the 632 plus bootstrap, and / or bagging (Hastie et al., 2001) applied to the naive Bayes classifier and the nearest shrunken centroid classifier (Tibshirani et al., 2002) and the support vector machine (SVM) which both performed well in classifying prostate tissues as malignant or benign, used in our previous work. The implementation likely to be used is to perform k-fold cross-validation. Within each of the k train/test cycles an initial screen of the training data for differentially expressed genes is performed and genes are ordered according to their posterior probability of differential expression. Naive Bayes and nearest shrunken centroid classifiers based on the r genes with the highest posterior probability of differential expression are formed choosing enough values of r between 1 and 1024 to allow accurate interpolation of the classification error rate. The "one se rule" (Brieman et al., 1984) is applied to the error rates for the test sets to choose the classifier that minimizes the error rate. For SVM, an internal 632+ bootstrap is applied to each training sample to select the number of genes to be used in forming the classifier. The "1 se rule" error rates from the k test sets are used to characterize the classification accuracy.

In addition to the use of univariate and multivariate statistical analysis tools, sophisticated bioinformatic analysis approaches will help make sense of possible biological links between the genes found to be differentially expressed between, *e.g*., melanoma and non-melanoma samples. These approaches will focus on the analysis of genetic networks and pathways (Edelman et al., 2006; Kong et al., 2006; and Pang et al., 2006) and have been implemented in software packages such as Ingenuity (on the world wide web at ingenuity.com) and MetaCore (on the world wide web at genego.com). The identification of the biological links between genes that emerge from a gene expression microarray analysis can help put into context the biological meaningfulness of their expression patterns as well as help reduce the set of differentially expressed genes to be represented on a diagnostic panel based on their biology. The end result of this analysis will be to define a candidate expression classifier that will be validated in future, larger clinical trials.

**QC metrics for RNA, amplified cDNA and microarray data.** Following informed consent, the suspicious pigmented lesion was tape stripped using EGIR and then biopsied as per standard of care, The resulting RNA isolated from the EGIR tape was amplified and profiled on the Affymetrix U133 plus 2.0 GeneChip. Microarray data were normalized by the GCRMA algorithm. To assure high quality of microarray data are generated, QC metrics were established for RNA, amplified cDNA and microarray data. The quality of RNA was assessed by capillary electrophoresis using the Experion system (Biorad, Hercule, CA) and RNA with at least one visible 18S rRNA was further processed for RNA amplification. The amplified cDNA was quantified by the Nanodrop system and quality of the amplified cDNA was also assessed by the Experion system. The yield of the amplified cDNAs greater than 5 mg and the average size distribution of the cDNAs greater than 750 nt were carried forward for microarray hybridization. Quality of the array data was further assessed using simpleaffy program in R and the array data with scaling factor less than 5.0 and % present call greater than 30% were used for further data analysis.

**Melanomas distinguished from dysplastic nevi and normal skin.** After passing the array data QC, 14 melanomas, 40 dysplastic nevi and 12 normal skin specimens were further analyzed. First, gene expression values less than 50 across all samples were filtered out and 16716 probesets were tested. These 16716 probesets were subjected to a statistical analysis for differentially expressed genes among melanomas, dysplastic: nevi and normal skin using ANOVA (p<0.05), multiple testing correction algorithm (Westafall and Young permutation) and false discover rate (FDR) of 0.05. As indicated above, of the original 117 genes, an 89 gene panel (Table 2) was found to be a potential melanoma classifier. Further testing identified a 5-gene classifier (Table 3), a 30-gene classifier (Table 4) that includes newly identified genes, a 20-gene classifier (Table 5) that includes newly identified genes, and a 19-gene classifier that includes newly identified genes, which may all be used to discriminate melanomas from atypical nevi. The genes and respective classifier panels were analyzed using the Prediction Analysis of Microarrays (PAM) software freely available from Stanford University (Stanford, CA).

The PAM software uses a modification of the nearest centroid method, which computes a standardized centroid for each class in a training set. This refers to the average gene expression for each gene in each class divided by the within-class standard deviation for that gene. Nearest centroid classification takes the gene expression profile of a new sample, and compares it to each of these class centroids. The class, whose centroid it is closest to, in squared distance, is the predicted class for that new sample.

These genes were all subjected to a hierarchical clustering analysis and the melanoma specimens grouped together and were clearly distinguished from dysplastic nevi and normal skin. In addition, there are three distinct classes of dysplastic nevi; one is grouped together with normal skin and the second one was in between normal skin and melanomas, while the third one was grouped together with melanomas. These data suggest stratum corneum RNA, harvested by tape stripping with EGIR, can be used to distinguish melanoma from dysplastic nevi in suspiciously pigmented lesions.

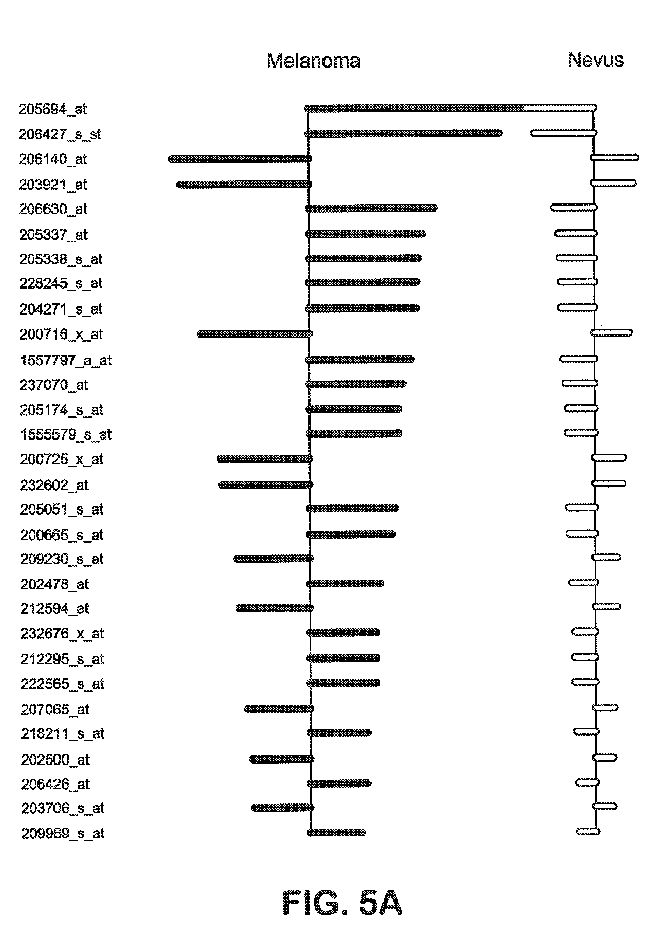

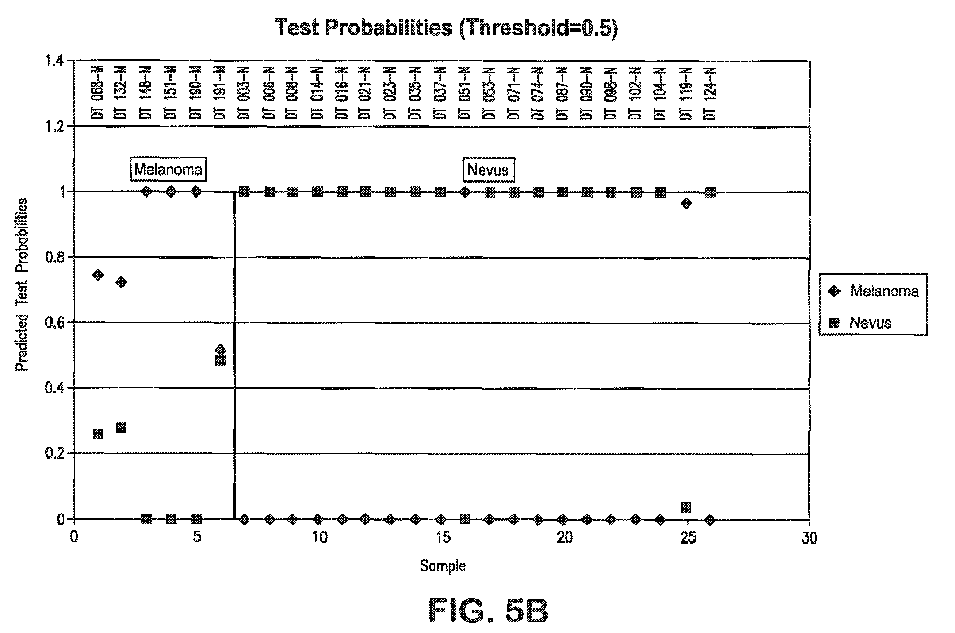

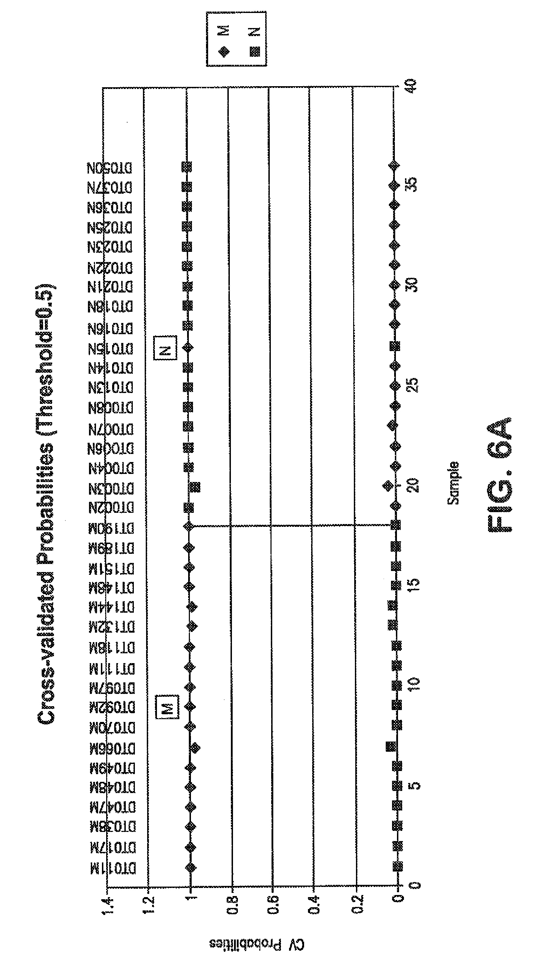

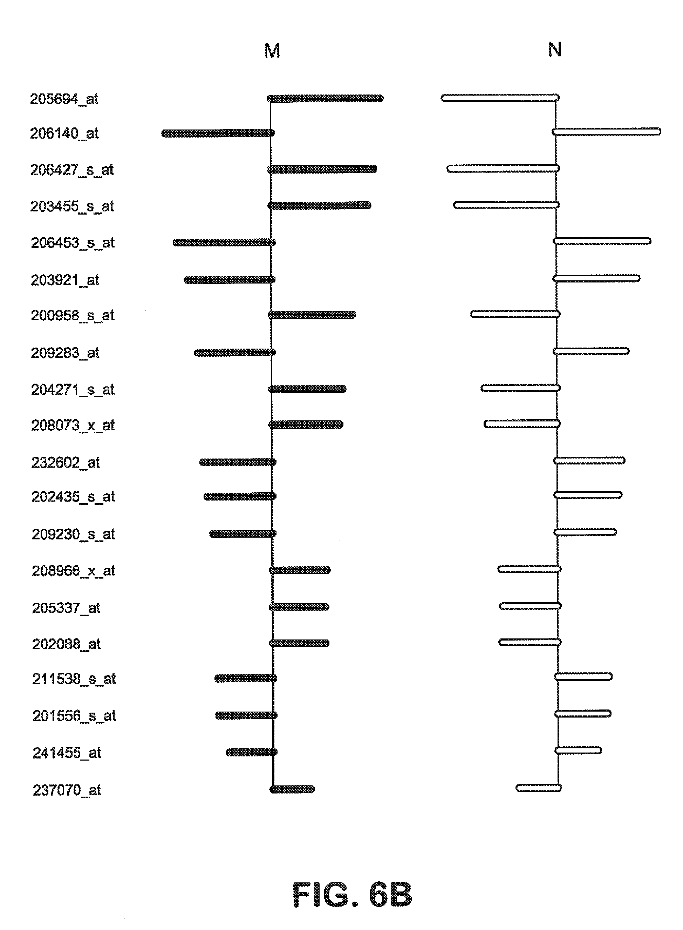

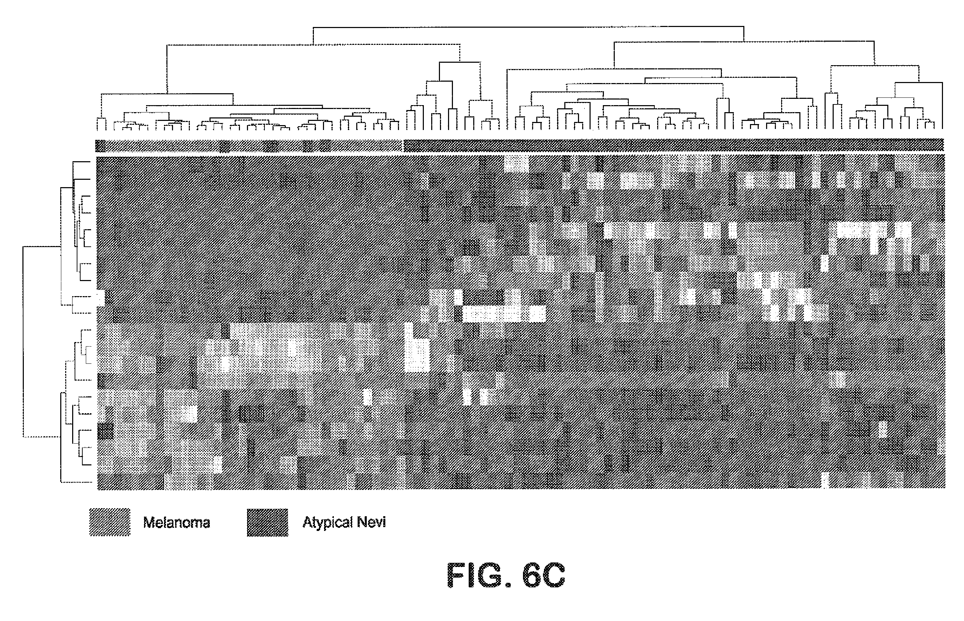

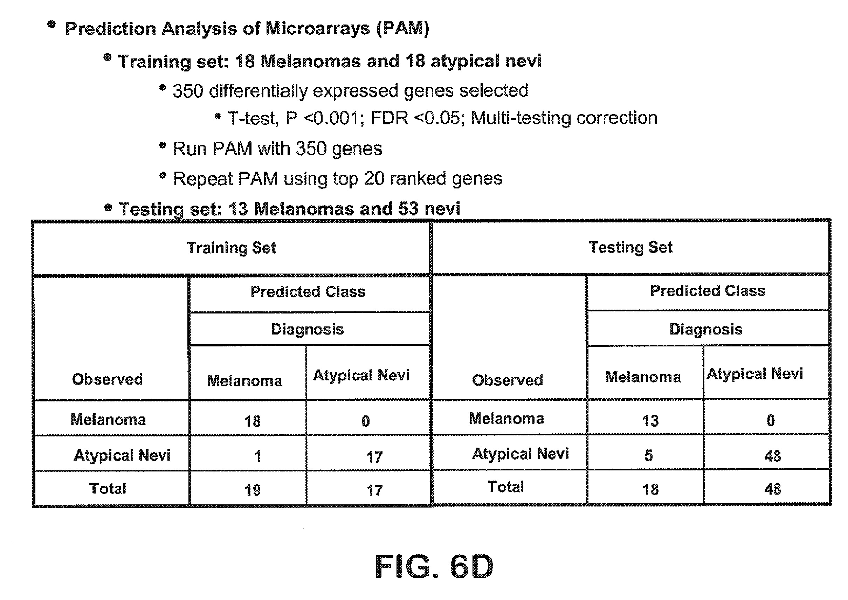

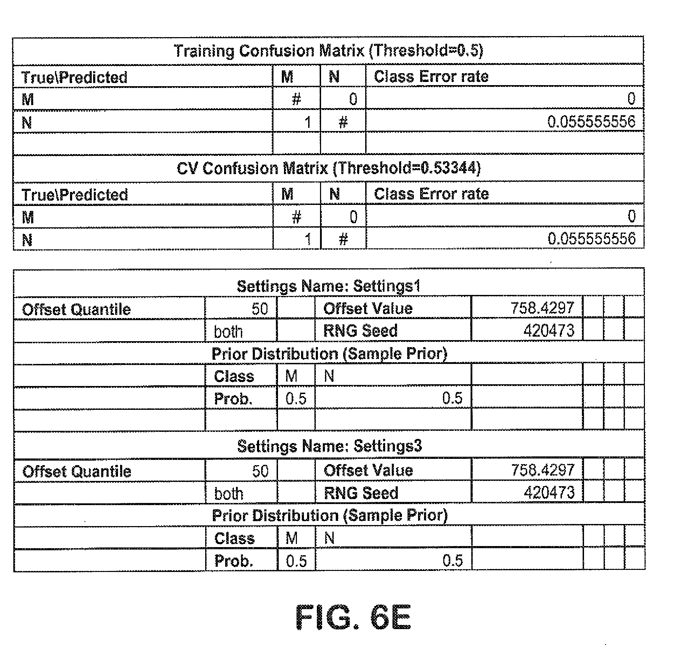

The analysis of the genes as potential melanoma classifiers to discriminate between melanomas and dysplastic nevi was performed using t-test (p<0.01), FDR (0.05) and 2-fold difference between melanomas and dysplastic nevi. Of the original 117 genes, an 89 gene panel (Table 2) was found to be a potential melanoma classifier and functions of these 89 genes were subjected to Ingenuity Pathway Analysis (IPA) (Ingenuity, Redwood City, CA). Among them, 15 genes are involved hair and skin development and function, 18 genes are involved in cellular development, 16 genes are involved in cellular growth and proliferation and 24 genes are related to cancer. Thus, differentially expressed genes are genes related to biological functions in melanocytes including melanin biosynthesis, melanocyte proliferation, differentiation and development. (See Figures 5 and 6).

### EXAMPLE 2

### Preliminary Power and Sample Size Studies: Nevi vs. Primary Melanoma

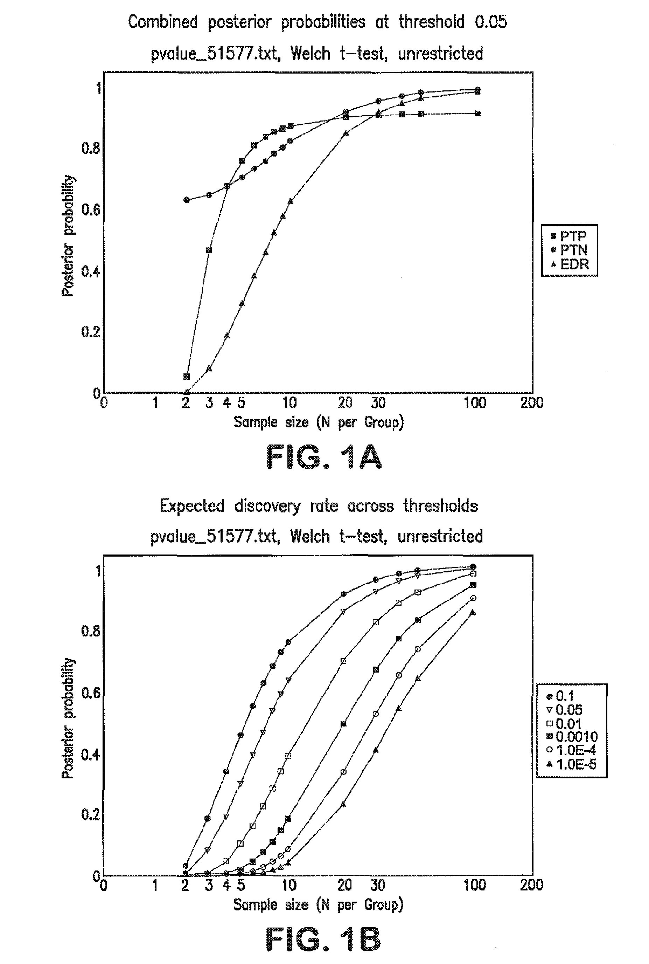

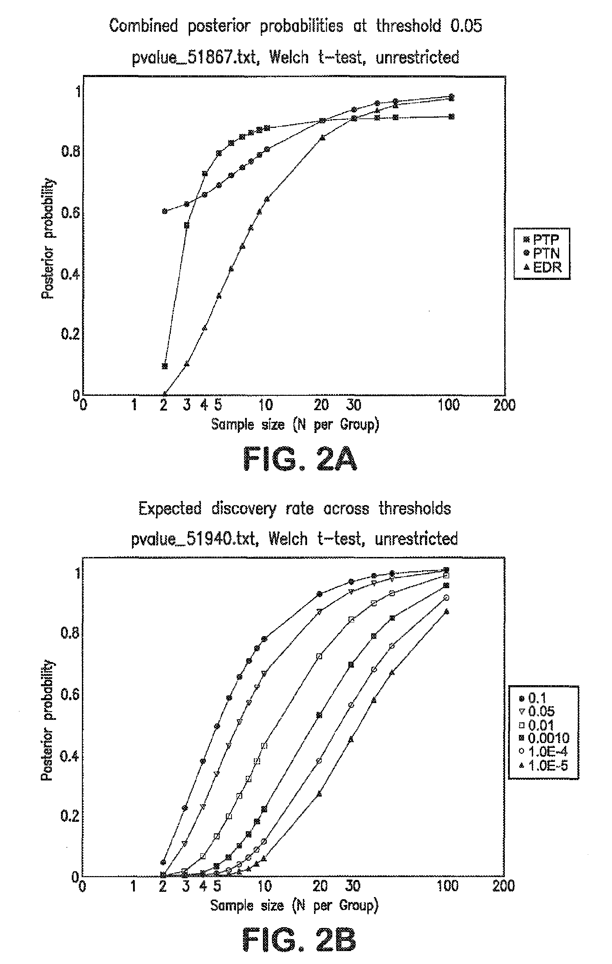

The following sample size and power calculations are based exclusively on the large-scale cDNA study data provided in Haqq et al (2005). That data focused on normal skin (n=3 samples), nevi (n=9), primary melanomas (n=6) and metastatic melanomas (n=19). For purposes of the sample size calculations, the focus was on the comparison of nevi to primary melanomas. Power and sample size assessments were calculated based on the bootstrap strategy outlined by Page et al. Using the raw data available from the Haqq et al (2005) study, gene expression differences—based on all 14,772 probes used in their cDNA assay-between nevi and primary melanomas were computed using simple t-tests for each probe/gene. Note that multiple probes can be used interrogate individual genes. In addition, normal skin, nevi, and primary melanoma gene expression differences were also assessed in a three group analysis of variance (ANOVA), with the specific contrast between nevi and primary melanoma computed from this ANOVA. In the figures that follow, three main parameters are used to assess power and sample size. Table 7 (adapted from Page, et al.) shows the number of genes truly or not truly differentially expressed, and provides a simple way of describing these parameters, which are defined as follows (with the color of the curves corresponding to each parameter provided in parentheses for Figures 1a and 2a, although Figures 1b and 2b focus exclusively on the EDR as defined below.

*EDR (Blue line):* Expected Discovery Rate (from Table 7, D / (B+D)). This reflects the expected proportion of probes/genes that will be declared significantly differentially expressed at the defined threshold (here taken to be, for the most part, p<0.05) that are, in fact, differentially expressed between nevi and primary melanomas.

*PTP (Red line):* Expected Proportion of probes/genes that are True Positives (Table 7, D / (C+D)). This proportion reflects the number of probes/genes showing expression differences that are likely to be truly differential expressed out of the total number of genes whose expression values result in test statistics less than the threshold (*e.g*., 0.05).

*PTN (Green line).* Probability of a True Negative result (Table 7, A / (A+B)). This probability concerns probes/genes that are not significantly different at the assumed threshold (*e.g*., 0.05) that are, in fact, not differentially expressed between skin and melanoma.

**Table 7. Parameters of Relevance for Assessing the Power of Microarray Studies**

| Result based on array analysis | Not differentially expressed | Truly differentially expressed |
|---|---|---|
| Genes not significant | A | B |
| Genes significant | C | D |

| | | |
|---|---|---|
| These columns represent the number of genes found to satisfy the given constraint; A = genes found not to be differentially expressed in an array experiment and that are truly not differentially expressed; B = genes that are differentially expressed but are not found to be differentially expressed in the array experiment (false negatives); C = genes that are found to be differentially expressed in the array experiment but are not truly differentially expressed (false positives); D = gene found to be differentially expressed in an array experiment and that are truly differentially expressed. | | |

**Nevi versus Primary data.** The sample size analysis considered the number of samples necessary to "discover" or identify a probe or gene or set of probes/genes that could differentiate nevi from primary melanomas based on the probe/gene expression differences obtained by Haqq et al. (2005). Figure 1a provides a plot of the EDR, PTP, and PTN as a function of sample size, assuming a threshold for declaring the significance of a probe/gene expression difference between nevi and primary melanoma of p < 0.05. Thus, from the plot, it appears that in order to "discover," or identify, 80% of all genes that have been interrogated on a chip that exhibit a probe/gene expression difference producing a test statistic p-value < 0.05 that will actually reflect a true probe/gene expression difference, a sample size of roughly 20 per nevi and primary melanoma group will be needed. Note that if all 14,772 probes are considered, one is likely to have 14,772 x 0.05 = 738 exhibit p-values < 0.05 by chance alone, of which 1,727 x 0.80 = 1,381 will likely reflect true gene expression differences at that significance (*i.e.*, p-value) level. If one is interested in identifying a smaller set of genes that have a greater probability of being detected as truly differentially expressed, a more stringent threshold for statistical significance (*e.g.,* 0.001) can be used. This would generate 14,772 x 0.001=15 genes with p-values < 0.001 by chance of which ∼45% (*i.e.,* 34 x 0.45 = 7 would likely be truly differentially expressed at that level; see Figure 1b; note curves in Figure 1b only correspond to the EDR with different assumed type I error rates).

A sample size analysis that considered the contrast results for nevi vs. primary melanoma in the context of an analysis of variance (ANOVA) comparing normal skin, nevi, and primary melanoma was also pursued. The rationale for this is that there are more differences between normal skin and either nevi or primary melanoma than there are between nevi and primary melanoma (based on an analysis of the Haqq et al (2005) data), and an analysis that considers normal skin gene expression variation may help reduce the noise in the assessment of nevi vs. primary melanoma gene expression differences. Figures 2a and 2b display the results of these analyses and provide similar sample size guidelines to those reflected in Figures 1a and 1b.

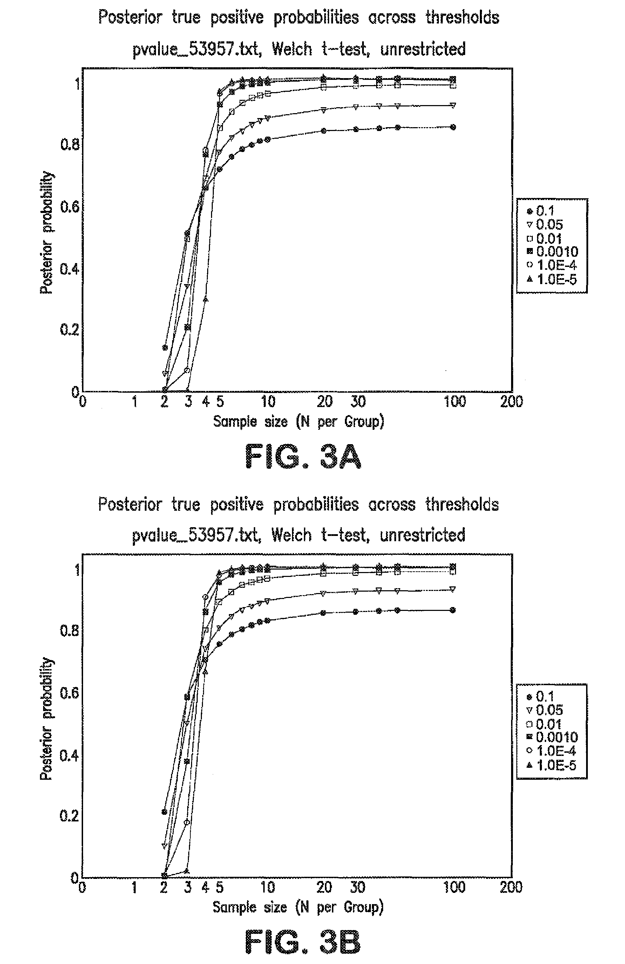

An analysis focusing exclusively on the posterior true probability (PTP) was also considered since, as discussed, there may be many probes/genes that exhibit differences between nevi and primary melanomas at a certain probability level purely by chance (given the large number of probes/genes interrogated). Thus, the likely fraction of these probes/genes that are truly differentially expressed is important to assess. Figures 3a and 3b reflect the results for different assumed significance levels.

Thus, an argument can be made that a study with approximately 20 samples per nevi and primary melanoma groups would have sufficient power to detect 80% of all genes that are likely to exhibit differential expression at a p-value level of 0.05 because they are, in fact, differentially expressed at this level. However, the number of genes (or probes) contributing to this set of differentially expressed genes is likely to number in the hundreds, if 10,000-30,000 probes are used or 5,000-10,000 genes are studied. If interest is in identifying a smaller number of probes or genes (∼25 - 40) that have a greater probability of being differentially expressed, say, at a p-value of 0.001, then ∼30 nevi and 30 primary melanoma samples would be needed (see Figures 1, 2, and 3).

### EXAMPLE 3

### Tape Stripping to Recover Nucleic Acids from Normal Skin

The following procedure was used to recover nucleic acids from normal skin (*e.g*., the mastoid or upper back areas) of a subject.

Tapes were handled with gloved hands at all times. Locate a particular site that is relatively blemish-free and healthy, unless otherwise specified by the protocol. Preferred normal skin sites are the mastoid process (the bony process behind the ear at the base of the skull) and the upper back, immediately superior to the scapular spine. Shave the site if necessary to remove non-vellus hairs. Cleanse the site with an alcohol wipe (70% isopropyl alcohol). Let the site air dry completely before application of the tape. It is recommended to wait approximately 2 minutes to ensure the site is completely dry before application of the tape.

Apply the tape to the skin site. If more than one tape is used, apply tapes in sequential order starting from the left side. Use a surgical skin marker and/or a water soluble marker to mark the location of the tape on the skin in order to align subsequent tapes.

Start the tape harvesting procedure by applying pressure (press on the tape firmly). Ensure that the skin is held taut to ensure that the tape does not move while applying pressure. Then remove the tape slowly in one direction. Place the edge of the tape onto the strip at the top of the packet with the adhesive surface of the tape facing down to protect the sample. Put a second tape on the same site; apply pressure firmly as above. Remove the tape slowly in an opposite direction to that used in the immediately previous application.

Continue tape stripping by putting additional tapes on the same site, following the steps provided above. The site may stripped with a total of at least four tapes, unless otherwise specified in the protocol. Place the strip into a storage bag and immediately place the samples on dry ice or into storage at -20°C or below until analysis.

### EXAMPLE 4

### Tape Stripping to Recover Nucleic Acids from Pigmented Lesions

The following procedure was used to recover nucleic acids from pigmented lesions and/or skin suspected of melanoma of a subject. In contrast to normal skin, lesional skin should have a preoperative biopsy diameter of greater than or equal to about 6 mm, but less than that of the tape disc- Multiple lesions must be at least about 4 mm apart. The area of tape that touches the lesion should be generously demarcated on the tape with an insoluble ink pen so that this area may be cut away from the surrounding tape at the laboratory as part of the RNA extraction procedure.

As above, tapes were handled with gloved hands at all times. Shave the site if necessary to remove non-vellus hairs. Cleanse the site with an alcohol wipe (70% isopropyl alcohol). Let the site air dry completely before application of the tape. It is recommended to approximately 2 minutes to ensure the site is completely dry before application of the tape.

Apply the tape to the skin site. If more than one tape is used, apply tapes in sequential order starting from the left side. Use a surgical skin marker and/or a water soluble marker to mark the location of the tape on the skin in order to align subsequent tapes. Apply the tape to the suspect lesion, which should have a diameter that is greater than or equal to about 6 mm.

Start the tape harvesting procedure by applying pressure directly over the lesion and avoiding surrounding normal skin (press on the tape firmly). Ensure that the skin is held taut to ensure that the tape does not move while applying pressure. Using a marking pen, demarcate a zone around the lesion such that the area of the lesion is encompassed within the inked boundary and the boundary is approximately 1 mm from the lesion border.

Remove the tape slowly in one direction. Place the edge of the tape onto the adhesive strip with cells facing down to protect the sample. Put a second tape on the same site following directions provided above. Repeat until the lesion has been stripped a total of at least four times, unless otherwise specified in the protocol. Place the strip into a storage bag and immediately place the samples on dry ice or into storage at -20°C or below until analysis.

### EXAMPLE 5

### Gene Expression Profile to Distinguish Melanoma from Atypical Nevi

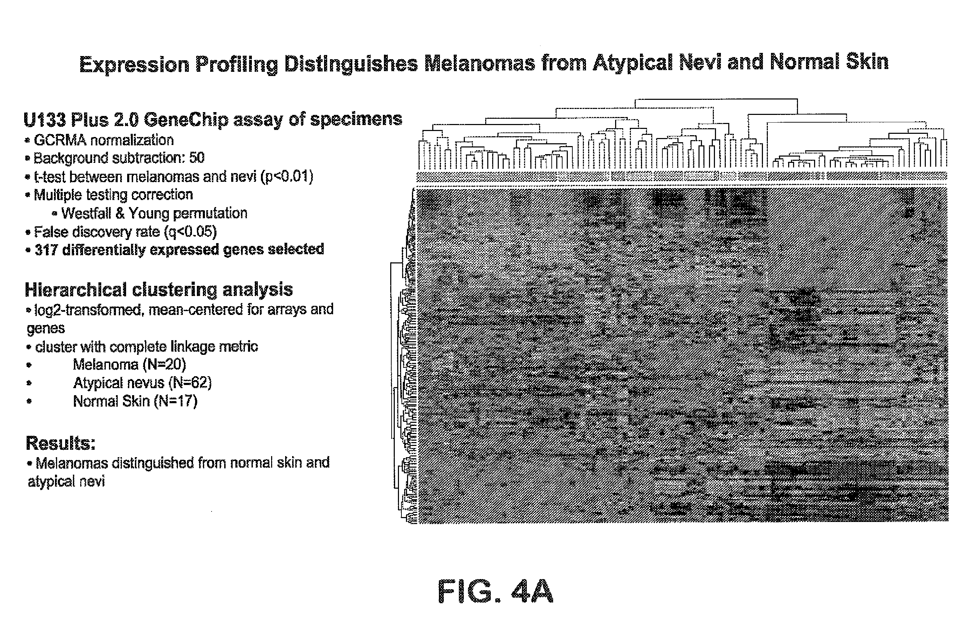

The purpose of this study is to determine whether stratum corneum RNA, harvested by tape stripping with EGIR can be used to distinguish melanoma from atypical nevi in suspicious pigmented lesions. See Figure 4A.

Suspicious pigmented lesions were tape stripped four times using EGIR and then biopsied as per standard of care. Normal, uninvolved skin was tape stripped to serve as a negative control. All biopsies underwent primary and central review for histopathology. Total RNA was isolated from the tapes using MELT (Ambion, Inc.) and assessed for quality by Experion (Bio-Rad, Inc.) analysis. The yield of RNA was approximately 1 ng, as determined by quantitative RT-PCR of the specimen for β-actin gene expression. Total RNA (200-500 pg) was then amplified using the WT-Ovation Pico RNA Amplification System (NuGen, Inc.) and assayed for gene expression profile using the U133 plus 2.0 GreneChip (Affymetrix, Inc.).

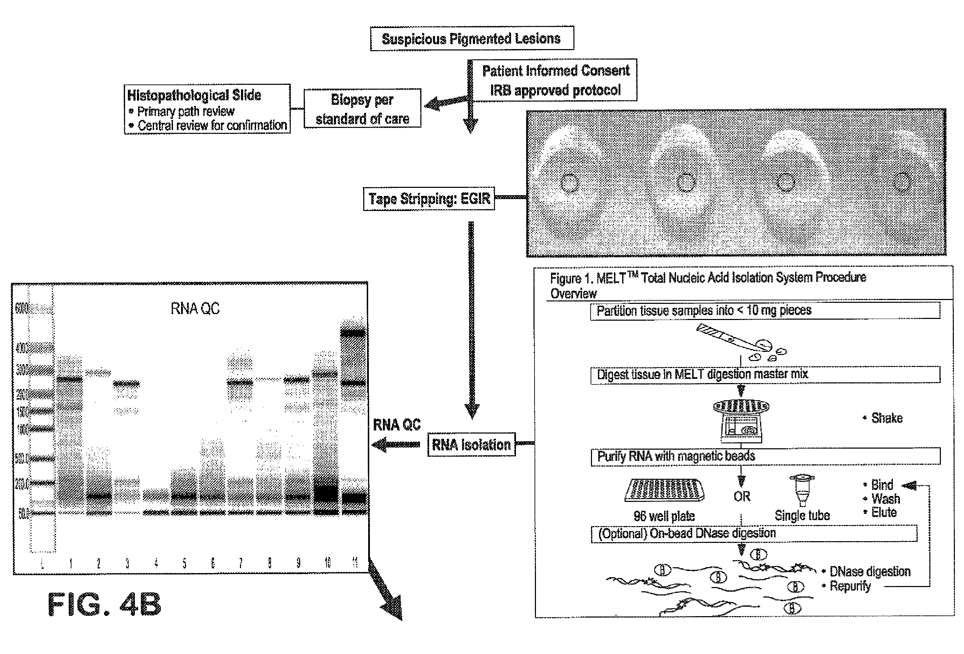

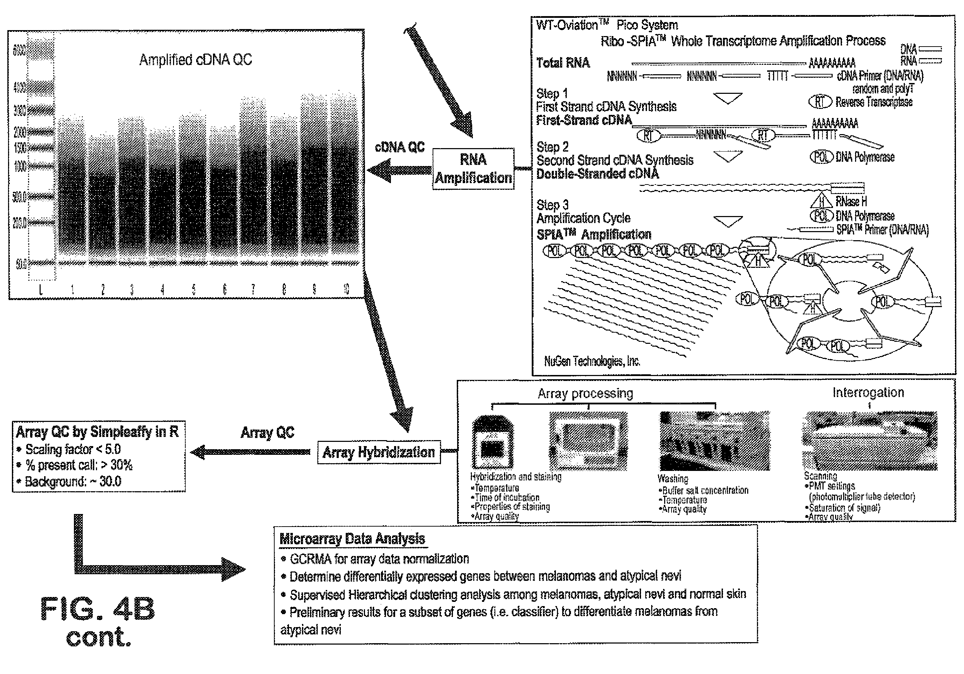

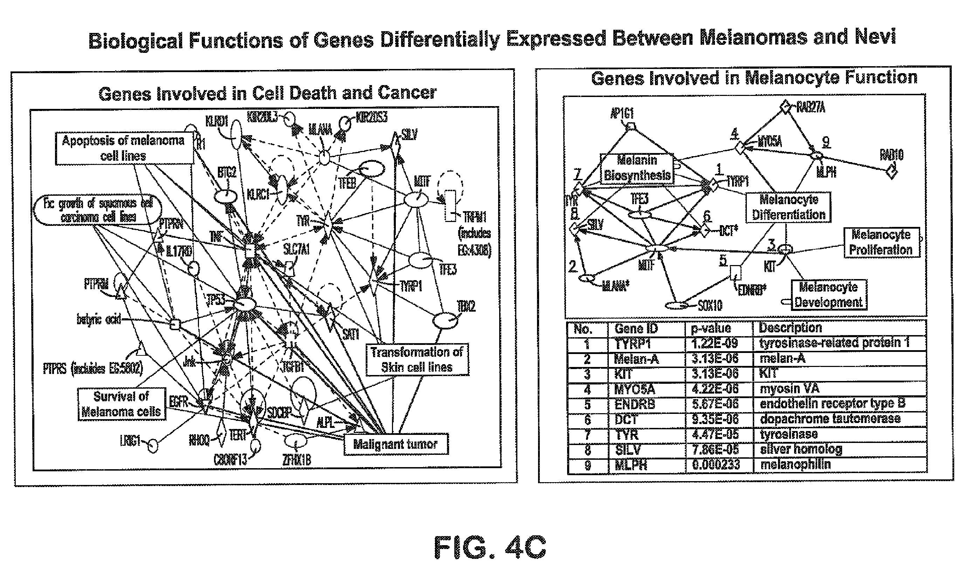

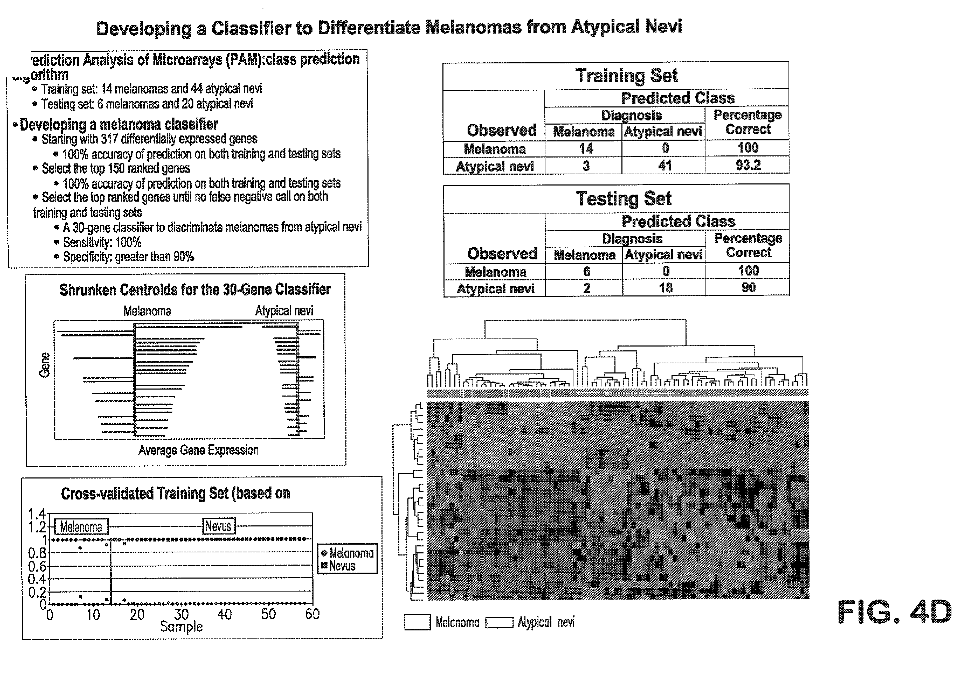

The resulting RNA isolated from the EGIR tape is then amplified and profiled on the Affymetrix U133 plus 2.0 GeneChip. Microarray data is normalized by the GCRMA algorithm. Further analyses by means of ANOVA analysis (p<0.05) with a false discovery rate of 0.05 and multiple correction testing using Westfall and Young permutation identified approximately 117 genes as differentially expressed between melanoma, dysplastic nevi and normal skin (Table 1). Hierarchical clustering of these genes showed that the melanoma specimens grouped together and were clearly distinguished from dysplastic nevi and normal skin (Figure 4B). In addition, 89 of the 117 genes shown in Table 1 were further identified (Table 2) as potential discriminators between melanoma and dysplastic nevi (p<0.01, false discovery rate q<0.05). When these 89 genes were subjected to Ingenuity Pathways analysis many were found to play roles in melanoma, hair and skin development and function, cellular development, cellular growth and proliferation and cancer. These findings demonstrate that EGIR-harvested RNA from suspicious pigmented skin lesions can be used to differentiate melanoma from dysplastic nevi (Figure 4C). Further, these results suggest that the gene expression profile of stratum corneum is altered, either directly or indirectly, by the presence of melanoma (Figure 4D).

In subsequent studies that compared normal and inflamed skin, sequential application of four small tapes at the same skin site recovered enough intact RNA to perform quantitative reverse-transcription polymerase chain reaction (qPCR) assay and DNA microarray analysis for investigation of gene expression. The latter assay was performed using the Affymetrix HG-U133A GeneChip following two rounds of amplification of 10 ng of total RNA sample that produced 30-80 µg of anti-sense RNA. Comparison of results from two subjects, each sampled at three separate sites, showed 12% intra- and inter-subject variance in gene measurements, a result that is well within the Affymetrix specified coefficient of variation (CV) for GeneChip assay. Of note is that differential expression of Y-chromosome genes was observed, a result that accurately distinguished the different genders of the 2 subject. GeneChip assay was then performed on RNA isolated from tape stripping each of 3 subjects from normal, water occluded, and sodium lauryl sulfate-irritated study groups. The majority of 100 genes, whose expression is most significantly altered between untreated and SLS-treated skin showed were already known to be involved in tissue inflammation and injury functions. Thus, RNA harvested by EGIR technology is more than adequate for microarray-based gene expression profiling and appropriately reflects the pathologic state of skin.

Recent work by Benson *et al* (2006) demonstrates that RNA can be recovered from psoriatic lesions and that the general RNA expression profile of tape strip recovered RNA is consistent with biopsy RNA derived from lesions on the same patient. Further work (see U.S. Pat. No. 7,183,057, incorporated herein by reference) has shown that psoriatic lesions can be sampled with tape during treatment with Enbrel and that strong correlations could be made between gene expression in week one of treatment and clinical response at weeks 4 and 8. This work further establishes the credentials of tape stripping for the recovery of physiologically relevant RNA from the surface of the skin.

### References

Jemal A, Murray T, Samuels A, Ghafoor A, Ward E, Thun MJ: Cancer statistics, 2003. CA Cancer J Clin 2003, 53(1):5-26.

Gloster HM, Jr., Brodland DG: The epidemiology of skin cancer. Dermatol Surg 1996, 22(3):217-226.

Albert VA, Koh HK, Geller AC, Miller DR, Prout MN, Lew RA: Years of potential life lost: another indicator of the impact of cutaneous malignant melanoma on society. JAm Acad Dermatol 1990, 23(2 Pt 1):308-310.

Morhenn VB, Chang EY, Rheins LA: A noninvasive method for quantifying and distinguishing inflammatory skin reactions. J Am Acad Dermatol 1999, 41(5 Pt 1):687-692.

Wong R, Tran V, Morhenn V, Hung SP, Andersen B, Ito E, Wesley Hatfield G, Benson NR: Use of RT-PCR and DNA microarrays to characterize RNA recovered by non-invasive tape harvesting of normal and inflamed skin. J Invest Dermatol 2004, 123(1):159-167.

Benson NR, Papenfuss J, Wong R, Motaal A, Tran V, Panko J, Krueger GG: An analysis of select pathogenic messages in lesional and non-lesional skin using non-invasive tape harvesting. Journal of Investigative Dermatology 2006, 126(10):2234-2241.

Baldi A, Santini D, De Luca A, Paggi MG: cDNA array technology in melanoma: an overview. J Cell Physiol 2003, 196(2):219-223.

Carr KM, Bittner M, Trent JM: Gene-expression profiling in human cutaneous melanoma. Oncogene 2003, 22(20):3076-3080.

Gershenwald JE, Bar-Eli M: Gene expression profiling of human cutaneous melanoma: are we there yet? Cancer Biol Ther 2004, 3(1):121-123.

Kim CJ, Reintgen DS, Yeatman TJ: The promise of microarray technology in melanoma care. Cancer Control 2002, 9(1):49-53.

Seftor RE, Seftor EA, Koshikawa N, Meltzer PS, Gardner LM, Bilban M, Stetler-Stevenson WG, Quaranta V, Hendrix MJ: Cooperative interactions of laminin 5 gamma2 chain, matrix metalloproteinase-2, and membrane type-1-matix/metalloproteinase are required for mimicry of embryonic vasculogenesis by aggressive melanoma. Cancer Res 2001, 61(17):6322-6327.

Su YA, Bittner ML, Chen Y, Tao L, Jiang Y, Zhang Y, Stephan DA, Trent JM: Identification of tumor-suppressor genes using human melanoma cell lines UACC903, UACC903(+6), and SRS3 by comparison of expression profiles. Mol Carcinog 2000, 28(2):119-127.

Haqq C, Nosrati M, Sudilovsky D, Crothers J, Khodabakhsh D, Pulliam BL, Federman S, Miller JR, 3rd, Allen RE, Singer MI et al: The gene expression signatures of melanoma progression. Proc Natl Acad Sci USA 2005, 102(17):6092-6097.

Paik S, Shak S, Tang G, Kim C, Baker J, Cronin M, Baehner FL, Walker MG, Watson D, Park T et al: A multigene assay to predict recurrence of tamoxifen-treated, node-negative breast cancer. N Engl J Med 2004, 351(27):2817-2826.

Pavey S, Johansson P, Packer L, Taylor J, Stark M, Pollock PM, Walker GJ, Boyle GM, Harper U, Cozzi SJ et al: Microarray expression profiling in melanoma reveals a BRAF mutation signature. Oncogene 2004, 23(23):4060-4067.

McCarty MF, Bielenberg DR, Nilsson MB, Gershenwald JE, Barnhill RL, Ahearne P, Bucana CD, Fidler IJ: Epidermal hyperplasia overlying human melanoma correlates with tumour depth and angiogenesis. Melanoma Res 2003, 13(4): 379-387.

Stolz W, Sehmoeckel C, Welkovich B, Braun-Falco O: Semiquantitative analysis of histologic criteria in thin malignant melanomas. J Am Acad Dermatol 1989, 20(6): 1115-1120.

Wu Z, Irizarry RA: Stochastic models inspired by hybridization theory for short oligonucleotide arrays. J Comput Biol 2005, 12(6):882-893.

Wu Z, Irizarry RA: Preprocessing of oligonucleotide array data. Nat Biotechnol 2004, 22(6):656-658; author reply 658.

Smyth GK, Yang YH, Speed T: Statistical issues in cDNA microarray data analysis. Methods Mol Biol 2003, 224:111-136.

Lee M-LT: Analysis of microarray gene expression data. Boston: Kluwer Academic Publishers; 2004.

Stuart RO, Wachsman W, Berry CC, Wang-Rodriguez J, Wasserman L, Klacansky I, Masys D, Arden K, Goodison S, McClelland M et al: In silico dissection of cell-type-associated patterns of gene expression in prostate cancer. Proc Natl Acad Sci U S A 2004, 101(2):615-620.

Mitchell R, Chiang CY, Berry C, Bushman F: Global analysis of cellular transcription following infection with an HIV-based vector. Mol Ther 2003, 8(4):674-687.

Wolyn DJ, Borevitz JO, Loudet O, Schwartz C, Maloof J, Ecker JR, Berry CC, Chory J: Light-response quantitative trait loci identified with composite interval and eXtreme array mapping in Arabidopsis thaliana. Genetics 2004, 167(2):907-917.

Borevitz JO, Liang D, Plouffe D, Chang HS, Zhu T, Weigel D, Berry CC, Winzeler E, Chory J: Large-scale identification of single-feature polymorphisms in complex genomes. Genome Res 2003, 13(3):513-523.

Efron B, Tibshirani R: Empirical bayes methods and false discovery rates for microarrays. Genet Epidemiol 2002, 23(1):70-86.

Hastie T, Tibshirani R, Friedman J: The elements of statistical learning: Date mining, inference, and prediction. New York: Springer-Verlag; 2001.

Tibshirani R, Hastie T, Narasimhan B, Chu G: Diagnosis of multiple cancer types by shrunken centroids of gene expression. Proc Natl Acad Sci USA 2002, 99(10):6567-6572.

Brieman L, Friedman J, Olshen R, Stone C: Classification and regression trees. Belmont, CA: Wadsworth International Group; 1984.

Edelman E, Porrello A, Guinney J, Balakumaran B, Bild A, Febbo PG, Mukherjee S: Analysis of sample set enrichment scores: assaying the enrichment of sets of genes for individual samples in genome-wide expression profiles. Bioinformatics 2006, 22(14):e108-116.

Kong SW, Pu WT, Park PJ: A multivariate approach for integrating genome-wide expression data and biological knowledge. Bioinformatics 2006.

Pang H, Lin A, Holford M, Enerson BE, Lu B, Lawton MP, Floyd E, Zhao H: Pathway analysis using random forests classification and regression. Bioinformatics 2006, 22(16):2028-2036.

Page GP, Edwards JW, Gadbury GL, Yelisetti P, Wang J, Trivedi P, Allison DB: The PowerAtlas: a power and sample size atlas for microarray experimental design and research. BMC Bioinformatics 2006, 7:84.

**Table 1**

| name | matched term | synonym | description | Entrez Gene ID for Human | Entrez Gene ID for Mouse | Entrez Gene ID for Rat |
|---|---|---|---|---|---|---|
| ACTR1B | 202135_s_at | 2310066K23Rik, | ARP1 actin-related protein 1 homolog B, centractin beta (yeast) | 10120 | 226977 | |
| (includes | | AA960180, | | | | |
| EG:10120) | | ACTR1B, | | | | |
| | | AI851923, | | | | |
| | | ARP1B, CTRN2, | | | | |
| | | MGC36526 | | | | |
| ANGEL1 | 213099_at | 1110030H02Rik, | angel homolog 1 | 23357 | 68737 | 362765 |
| | | KIAA0759, | (Drosophila) | | | |
| | | mKIAA0759, | | | | |
| | | RGD1306238 | | | | |
| ANKRD13B | 227720_at | AW124583, | ankyrin repeat domain 13B | 124930 | 268445 | 360575 |
| | | B930093C12Rik, | | | | |
| | | FLJ20418, | | | | |
| | | FLJ25555, | | | | |
| | | RGD1564005 | | | | |
| ANKRD44 | 228471_at | 4930444A19Rik, | ankyrin repeat domain 44 | 91526 | 329154 | 301415 |
| | | A130096K20, | | | | |
| | | E130014H08Rik, | | | | |
| | | LOC91526, | | | | |
| | | MGC21968, | | | | |
| | | MGC70444, | | | | |
| | | RGD1561893 | | | | |
| | 226857_at | 6030432F23, | Rho guanine nucleotide exchange factor (GEF) 19 | 128272 | 213649 | 362648 |
| ARHGEF19 | | 6430573B13Rik, | | | | |
| | | FLJ33962, RP4- | | | | |
| | | 733M16.1, | | | | |
| | | WGEF | | | | |
| ATPBD4 | 238662_at | 5730421E18Rik, | ATP binding domain 4 | 89978 | 66632 | 362191 |
| | | MGC14798, | | | | |
| | | RGD1310006 | | | | |
| BARX2 | 210419_at | 2310006E12Rik, | BarH-like homeobox 2 | 8538 | 12023 | |
| | | Barx2b, | | | | |
| | | MGC133368, | | | | |
| | | MGC133369 | | | | |
| BDNF | 206382_s_at | MGC105254, | brain-derived | 627 | 12064 | 24225 |
| | | MGC34632 | neurotrophic factor | | | |
| BLOCISI | 202592_at | AI839753, | biogenesis of lysosome-related organelles | 2647 | 14533 | 288785 |
| | | BLOC-1 subunit | | | | |
| | | 1, BLOS1, | complex-1, subunit 1 | | | |
| | | GCN5-like | | | | |
| | | protein 1, | | | | |
| | | GCN5L1, | | | | |
| | | MGC87455, | | | | |
| | | RT14 | | | | |
| BTG2 | 201236_s_at | AA959598, Agl, | BTG family, member 2 | 7832 | 12227 | 29619 |
| | | An, an-1, | | | | |
| | | APRO1, | | | | |
| | | MGC 126063, | | | | |
| | | MGC126064, | | | | |
| | | PC3, TIS21 | | | | |
| C160RF48 | 223407_at | AI606951, | chromosome 16 open reading frame 48 | 84080 | 102124 | 291975 |
| | | DAKV6410, | | | | |
| | | DKFZP434A131 | | | | |
| | | 9, | | | | |
| | | E130303B06Rik, | | | | |
| | | RGD1307357 | | | | |
| C6ORF218 | 244829_at | MGC40222 | chromosome 6 open reading frame 218 | 221718 | | |
| | | | | | | |
| C8ORF13 | 233641_s_at | A030013D21, | chromosome 8 open reading frame 13 | 83648 | 219148 | 498533 |
| | | BC065085, | | | | |
| | | D8S265, | | | | |
| | | DKFZp761G151, | | | | |
| | | MGC120649, | | | | |
| | | MGC120650, | | | | |
| | | MGC120651, | | | | |
| | | RGD1561302 | | | | |
| CCDC95 | 227286_at | AI225782, | coiled-coil domain containing 95 | 283899 | 233875 | |
| | | AI854876, | | | | |
| | | Ccdc85, | | | | |
| | | FLJ00079, | | | | |
| | | FLJ90652, | | | | |
| | | MGC31515 | | | | |
| CCHCR1 | 37425_g_at | C6orf18, HCR, | coiled-coil alpha-helical rod protein 1 | 54535 | 240084 | 406196 |
| | | MGC126371, | | | | |
| | | MGC126372, | | | | |
| | | MGC28303, | | | | |
| | | RGD:1302992, | | | | |
| | | SBP | | | | |
| CIRBP | 230142_s_at | A18 HNRNP, | cold inducible RNA | 1153 | 12696 | 81825 |
| | | CIRP, R74941 | binding protein | | | |
| CLSTN2 | 219414_at | 2900042C18Rik, | calsyntenin 2 | 64084 | 64085 | 171394 |
| | | AI448973, | | | | |
| | | alcagamma, CS2, | | | | |
| | | Cst-2, CSTN2, | | | | |
| | | FLJ39113, | | | | |
| | | FLJ39499, | | | | |
| | | KIAA4134, | | | | |
| | | MGCD9560, | | | | |
| | | mKIAA4134 | | | | |
| COL7A1 | 217312_s_at | AW209154, | collagen, type VII, alpha 1 (epidermolysis bullosa, dystrophic, dominant and recessive) | 1294 | 12836 | 301012 |
| | | EBD1, EBDCT, | | | | |
| | | EBR1 | | | | |
| DACH1 | 205471_s_at, | AI182278, Dac, | dachshund homolog 1 (Drosophila) | 1602 | 13134 | |
| | 205472_s_at, | DACH, | | | | |
| | 228915_at | E130112M23Rik, | | | | |
| | | FLJ10138 | | | | |
| DCT | 205337_at, | DT, | dopachrome tautomerase (dopachrome delta-isomerase, tyrosine-related protein 2) | 1638 | 13190 | 290484 |
| | 205338_s_at | RGD1564975, | | | | |
| | | slaty, sIt, TRP-2, | | | | |
| | | TYRP2 | | | | |
| DOCK10 | 219279_at | | dedicator of cytokinesis 10 | 55619 | 210293 | 301556 |
| | | 9330153B10RIK, | | | | |
| | | A630054M16Rik | | | | |
| | | | | | | |
| | | DKFZp781A153 | | | | |
| | | 2, DRIP2, Jr4, | | | | |
| | | Jr5, mKIAA0694, | | | | |
| | | NbIa10300, | | | | |
| | | R75174, | | | | |
| | | RGD1561963, | | | | |
| | | ZIZ3, Zizimin3 | | | | |
| DRAP1 | 1556181_at | 2310074H19Rik, | DR1-associated protein 1 (negative cofactor 2 alpha) | 10589 | 66556 | 293674 |
| | | MGC156767, | | | | |
| | | NC2-ALPHA, | | | | |
| | | negative cofactor | | | | |
| | | 2 alpha | | | | |
| EDNRB | 204271_s_at, | ABCDS, | endothelin receptor type B | 1910 | 13618 | 50672 |
| | 206701_x_at | AU022549, | | | | |
| | | Ednra, | | | | |
| | | ET>B<, | | | | |
| | | ET-B, ETB | | | | |
| | | RECEPTOR, | | | | |
| | | ETBR, ETRB, | | | | |
| | | GUSB, HSCR, | | | | |
| | | HSCR2, | | | | |
| | | Sox10m1 | | | | |
| EFNA4 | 205107_s_at | EFL-4, EPHRIN | ephrin-A4 | 1945 | 13639 | 310643 |
| | | A4, Ep14, | | | | |
| | | EPLG4, LERK-4, | | | | |
| | | MGC125826 | | | | |
| EHD2 | 45297_at | BC027084, | EH-domain containing 2 | 30846 | 259300 | 361512 |
| | | C130052H20Rik, | | | | |
| | | MGC25606, | | | | |
| | | MGC38650, | | | | |
| | | MGEPS, PAST2 | | | | |
| ETS1 | 224833_at | AI196000, | v-ets erythroblastosis virus E26 oncogene homolog I (avian) | 2113 | 23871 | 24356 |
| | | AI448617, C- | | | | |
| | | ETS1, | | | | |
| | | D230050P06, | | | | |
| | | Etsoncb, EWSR2, | | | | |
| | | FLJ10768, | | | | |
| | | MGC124638, | | | | |
| | | MGG130355, | | | | |
| | | MGC18571, p42 | | | | |
| | | ETS1, p51 ETS1, | | | | |
| | | TpI1 | | | | |
| FAM33A | 225684_at | 1110001A07Rik, | family with sequence similarity 33, member A | 348235 | | 287598 |
| | | C78640, | | | 66140\|62 | |
| | | EG625534, | | | 5534 | |
| | | FLJ12758, | | | | |
| | | MGC109093, | | | | |
| | | MGC110975, | | | | |
| | | MGC151378, | | | | |
| | | RGD1307084 | | | | |
| FGFR1 | 210973_s_at, | AW208770, | fibroblast growth factor receptor 1 (fms-related tyrosine kinase 2, Pfeiffer syndrome) | 2260 | 14182 | 79114 |
| | 211535_s_at | BFGFR, C-FGR, | | | | |
| | | CD331, CEK, | | | | |
| | | FGF 1 | | | | |
| | | RECEPTOR, | | | | |
| | | FGFBR, FGFR1- | | | | |
| | | IIIC, Fgfr1c, | | | | |
| | | FLG, Flk2, FLT2, | | | | |
| | | H5, HBGFR, | | | | |
| | | KAL2, N-SAM | | | | |
| FOXO1A | 202723_s_at | Afxh, AI876417, | forkhead box O1A (rhabdomyosarcoma) | 2308 | 56458 | 84482 |
| | | FKH1, FKHR, | | | | |
| | | FKHR1, | | | | |
| | | Forkhead, | | | | |
| | | FOXO1 | | | | |
| FOXP1 | 223936_s_at | 12CC4, | forkhead box P1 | 27086 | 108655 | 297480 |
| | | 3110052D19Rik, | | | | |
| | | 4932443N09Rik, | | | | |
| | | AI461938, | | | | |
| | | AW494214, | | | | |
| | | FLJ23741, | | | | |
| | | hFKH1B, | | | | |
| | | HSPC215, | | | | |
| | | MGC116362, | | | | |
| | | MGC12942, | | | | |
| | | MGC88572, | | | | |
| | | MGC99551, | | | | |
| | | QRF 1 | | | | |
| FRAT2 | 209864_at | MGC10562, | frequently rearranged in advanced T-cell lymphomas 2 | 23401 | 212398 | |
| | | MGC37615 | | | | |
| | | | | | | |
| GCLM | 203925_at | Gamma gcIm, | glutamate-cysteine ligase, modifier subunit | 2730 | 14630 | 29739 |
| | | Gamma | | | | |
| | | glutamylcysteine | | | | |
| | | synthase | | | | |
| | | (regulatory), | | | | |
| | | GAMMA | | | | |
| | | GLUTAMYLCY | | | | |
| | | STEINE | | | | |
| | | SYNTHETASE, | | | | |
| | | Gcs Ls, Gcs, | | | | |
| | | Regulatory, GCS- | | | | |
| | | L, GCS1, Gcslc, | | | | |
| | | GLCLR, | | | | |
| | | gtutamat-cystein | | | | |
| | | ligase, regulatory | | | | |
| | | subunit | | | | |
| GGA3 | 209411_s_at | | golgi associated, gamma adaptin ear containing, ARF binding protein 3 | 23163 | 260302 | 360658 |
| | | C230037M19Rik, | | | | |
| | | KIAA0154, | | | | |
| | | mKIAA0154 | | | | |
| GLUL | 200648_s_at | GLNS, | glutamate-ammonia | 2752 | 14645 | |
| | | Glutamine | ligase (glutamine | | | |
| | | Synthase, | synthetase) | | | |
| | | GLUTAMINE | | | | |
| | | SYNTHETASE, | | | | |
| | | GS, | | | | |
| | | MGC128403, | | | | |
| | | PIG43 | | | | |
| GPR161 | 214104_at | FLJ33952, | G protein-coupled receptor 161 | 23432 | 240888 | 289180 |
| | | G-protein coupled | | | | |
| | | receptor | | | | |
| | | af091890, | | | | |
| | | Gm208, | | | | |
| | | Gm208Gpr, RE2, | | | | |
| | | RGD1563245 | | | | |
| HEY2 | 219743_at | CHF1, GRL, | hairy/enhancer-of-split related with YRPW motif 2 | 23493 | 15214 | 155430 |
| | | HERP1, HESR2, | | | | |
| | | HRT2, | | | | |
| | | MGC10720 | | | | |
| HIST2H2AA3 | 214290_s_at | AI448581, H2A, | histone cluster 2, H2aa3 | 8337 | 15267 | 365877 |
| | | H2a-615, H2A.2, | | | | |
| | | H2A/O, H2A/q, | | | | |
| | | H2AFO, Hist2, | | | | |
| | | HIST2H2AA, | | | | |
| | | Hist2h2aa1 | | | | |
| ID1 | 208937_s_at | AI323524, | inhibitor of DNA binding 1, dominant negative helix-loop-helix protein | 3397 | 15901 | 25261 |
| | | D2Wsu140e, ID, | | | | |
| | | ID-1H, ID125A, | | | | |
| | | IdbI, | | | | |
| | | MGC 156482 | | | | |
| KALRN | 227750_at | 2210407GI4Rik, | kalirin, RhoGEF kinase | 8997 | 545156 | 84009 |
| | | AV235988, | | | | |
| | | DUET, Duo, | | | | |
| | | E530005C20Rik, | | | | |
| | | FLJ16443, | | | | |
| | | Gm539, HAPIP, | | | | |
| | | KALIRIN, | | | | |
| | | Kalirin7, Pcip10, | | | | |
| | | TRAD | | | | |
| KDELR1 | 200922_at | 8030486F04Rik, | KDEL (Lys-Asp-Glu-Leu) endoplasmic reticulum protein retention receptor I | 10945 | 68137 | 361577 |
| | | AW215843, | | | | |
| | | ERD2, ERD2.1, | | | | |
| | | HDEL, KDEL | | | | |
| | | RECEPTOR, | | | | |
| | | Kdelr, | | | | |
| | | MGC109169, | | | | |
| | | PM23 | | | | |
| KIAA0738 | 210529_s_at | 2810407D09Rik, | KIAA0738 gene product | 9747 | 77574 | 362353 |
| | | 3321401G04Rik, | | | | |
| | | A230020K05Rik, | | | | |
| | | AI848529, | | | | |
| | | RGD1565474 | | | | |
| KIT | 205051_s_at | Bs, C-KIT, c-Kit | v-kit Hardy-Zuckerman 4 feline sarcoma viral oncogene homolog | 3815 | 16590 | 64030 |
| | | Gnnk+, CD117, | | | | |
| | | Fdc, SCFR, Ssm, | | | | |
| | | Tr Kit, white-spotted | | | | |
| LGR4 | 230674_at | 9130225G07, | leucine-rich repeat-containing G protein-coupled receptor 4 | 55366 | 107515 | 286994 |
| | | A930009A08Rik, | | | | |
| | | GPCR48, GPR48 | | | | |
| LHX2 | 211219_s_at | ap, apterous, | LIM homeobox 2 | 9355 | 16870 | 296706 |
| (includes | | hLhx2, Lh-2, | | | | |
| EG:9355) | | LH2A, Lhx2, | | | | |
| | | Lim2, | | | | |
| | | MGC138390 | | | | |
| LMO4 | 209204_at | | LIM domain only 4 | 8543 | 16911 | 362051 |
| | | A730077C12Rik, | | | | |
| | | Crp3, Etohi4, | | | | |
| | | MGC105593 | | | | |
| LOC254100 | 1557131_at | | hypothetical protein LOC254100 | 254100 | | |
| | | LOC254100 | | | | |
| LRIG1 | 236173_s_at, | D6Bwg0781e, | leucine-rich repeats and immunoglobulin-like domains I | 26018 | 16206 | 312574 |
| | 238339_x_at | DKFZP586O162 | | | | |
| | | 4, Img, LIG-1 | | | | |
| MED28 | 222635_s_at | 1500003D12Rik, | mediator of RNA polymerase II transcription, subunit 28 homolog (S. cerevisiae) | 80306 | 66999 | 305391 |
| | | AI451633, | | | | |
| | | AU045690, | | | | |
| | | DKFZP434N185, | | | | |
| | | EG1, FKSG20, | | | | |
| | | magicin, | | | | |
| | | RGD1305875 | | | | |
| MKL1 | 215292_s_at | AI852829, | megakaryoblastic leukemia (translocation) 1 | 57591 | 223701 | 315151 |
| | | AMKL, | | | | |
| | | AW743281, | | | | |
| | | AW821984, | | | | |
| | | BSAC, MAL, | | | | |
| | | MRTF-A | | | | |
| MLANA | 206426_at, | A930034P04Rik, | melan-A | 2315 | 77836 | 293890 |
| | 206427_s_at | MART-1, | | | | |
| | | MELAN-A, | | | | |
| | | MGC130556 | | | | |
| MLLT6 | 225628_s_at | AF17, | myeloid/lymphoid or mixed-lineage leukemia (trithorax homolog, Drosophila); translocated to, 6 | 4302 | 246198 | 303504 |
| | | AI315037, | | | | |
| | | FLJ23480 | | | | |
| MLPH | 218211_s_at | 22I0418F23Rik, | melanophilin | 79083 | 171531 | 316620 |
| | | 5031433I09Rik, | | | | |
| | | AW228792, | | | | |
| | | D1 Wsu84e, 1(1)- | | | | |
| | | 3Rk, 11Rk3, In, | | | | |
| | | MGC2771, | | | | |
| | | MGC59733, | | | | |
| | | SLAC2-A | | | | |
| MYEF2 | 222771_s_at, | 9430071B01, | myelin expression factor 2 | 50804 | 17876 | 362207 |
| | 232676_x_at | FLJ11213, | | | | |
| | | HsT18564, | | | | |
| | | KIAA1341, | | | | |
| | | MEF-2, | | | | |
| | | MGC109392, | | | | |
| | | MGC87325, | | | | |
| | | mKIAA1341, | | | | |
| | | MST156, | | | | |
| | | MSTP156 | | | | |
| MYL6B | 204173_at | 5730437E04Rik, | myosin, light chain 6B, alkali, smooth muscle and non-muscle | 140465 | 216459 | 317454 |
| | | Myosin | | | | |
| | | Light Chain 1, | | | | |
| | | BC037527, | | | | |
| | | MGC41229, | | | | |
| | | MLC1SA, | | | | |
| | | RGD1560334 | | | | |
| MYO5A | 227761_at | 9630007J19Rik, | myosin VA (heavy chain 12, myoxin) | 4644 | 17918 | 25017 |
| | | AI413174, | | | | |
| | | AI661011, Br | | | | |
| | | Myosin5a, d- | | | | |
| | | 120J, Dbv, Dop, | | | | |
| | | flail, flr, GS1, | | | | |
| | | hcBM-V, MVa, | | | | |
| | | MYH12, MYO5, | | | | |
| | | myosin V, | | | | |
| | | MYOSIN VA, | | | | |
| | | MYOSIN VA | | | | |
| | | EXON | | | | |
| | | CONTAINING, | | | | |
| | | MYOVA, | | | | |
| | | MYOXIN, | | | | |
| | | MYR12, Sev-1 | | | | |
| NBL1 | 37005_at | D1S1733E, | neuroblastoma, suppression of tumorigenicity 1 | 4681 | 17965 | 50594 |
| | | D4H1S1733E, | | | | |
| | | DAN, Dana, | | | | |
| | | DAND1, | | | | |
| | | MGC123430, | | | | |
| | | MGC8972, NB, | | | | |
| | | NO3 | | | | |
| NFIB | 230791_at | 6720429L07Rik, | nuclear factor IB | 4781 | 18028 | 29227 |
| | | CTF/NF1B, | | | | |
| | | E030026I10Rik, | | | | |
| | | NF1-B, NFI- | | | | |
| | | RED, NFIB2, | | | | |
| | | NFIB3, Nuclear | | | | |
| | | factor 1/B | | | | |
| OSTM1 | 218196_at | 1200002H13Rik, | osteopetrosis associated transmembrane protein 1 | 28962 | 14628 | 445370 |
| | | AW123348, | | | | |
| | | GIPN, GL, | | | | |
| | | HSPC019 | | | | |
| PDK3 | 221957_at | | pyruvate dehydrogenase kinase, isozyme 3 | 5165 | 236900 | 296849 |
| | | 2610001M10Rik, | | | | |
| | | AI035637, | | | | |
| | | MGC6383 | | | | |
| PKD1 | 241090_at | FLJ00285, | polycystic kidney disease 1 (autosomal dominant) | 5310 | 18763 | 24650 |
| | | mFLJ00285, | | | | |
| | | MGC118471, | | | | |
| | | PBP, PC-1, | | | | |
| | | POLYCYSTIN1 | | | | |
| PLEKHA5 | 220952_s_at | 2810431N21Rik, | pleckstrin homology domain containing, family A member 5 | 54477 | 109135 | 246237 |
| | | AI428202, | | | | |
| | | AK129423, | | | | |
| | | Ayu21-9, | | | | |
| | | FLJ10667, | | | | |
| | | FLJ31492, | | | | |
| | | Gt(pU21)9Imeg, | | | | |
| | | Image:3710928, | | | | |
| | | KIAA1686, | | | | |
| | | MGC38455, | | | | |
| | | PEPP2, TRS1 | | | | |
| PLP1 | 210198_s_at | DM20, jimpy, jp, | proteolipid protein 1 | 5354 | 18823 | 24943 |
| | | MMPL, Msd, | (Pelizaeus-Merzbacher disease, spastic paraplegia 2, uncomplicated) | | | |
| | | PLP, PLP/DM20, | | | | |
| | | PMD, | | | | |
| | | PROTEOLIPID, | | | | |
| | | RSH, SPG2 | | | | |
| PLXNC1 | 213241_at | 2510048K12Rik, | plexin C1 | 10154 | 54712 | 362873 |
| | | AW742158, | | | | |
| | | CD232, Plexin | | | | |
| | | C1, VESPR | | | | |
| PPP3CA | 202425_x_at | 2900074D19Rik, | protein phosphatase 3 | 5530 | 19055 | 24674 |
| | | AI841391, | (formerly 2B), catalytic subunit, alpha isoform (calcineurin A alpha) | | | |
| | | AW413465, | | | | |
| | | Calcineurin, | | | | |
| | | Calcineurin A | | | | |
| | | Alpha, CALN, | | | | |
| | | CALNA, | | | | |
| | | CALNAI, CCNI, | | | | |
| | | CN, CnA, CnA- | | | | |
| | | alpha, CNA1, | | | | |
| | | MGC106804, | | | | |
| | | Pp2b Subunit A, | | | | |
| | | PPP2B | | | | |
| PRKCSH | 200707_at | 80K-H, AGE- | protein kinase C substrate 80K-H | 5589 | 19089 | 300445 |
| | | R2, G19P1, | | | | |
| | | PCLD, PLD, | | | | |
| | | PLD1 | | | | |
| PRKD3 | 222565_s_at | 4930557O20Rik, | protein kinase D3 | 23683 | 75292 | 313834 |
| | | 5730497N19Rik, | | | | |
| | | EPK2, | | | | |
| | | MGC47171, | | | | |
| | | nPKC-NU, PKC- | | | | |
| | | NU, PKD3, | | | | |
| | | PRKCN | | | | |
| NPRM1 | 206445_s_at | 6720434D09Rik, | protein arginine | 3276 | 15469 | 60421 |
| | | ANM1, | methyltransferase 1 | | | |
| | | AW214366, | | | | |
| | | HCP1, | | | | |
| | | heterogeneous | | | | |
| | | ribonucleooprotei | | | | |
| | | ns | | | | |
| | | methyltransferase -like 2, Hnmt112, | | | | |
| | | Hramt, | | | | |
| | | HRMT1L2, | | | | |
| | | IR1B4, Mrmt1 | | | | |
| PSCD3 | 225147_at | AI648983, | pleckstrin homology, Sec7 and coiled-coil domains 3 | 9265 | 19159 | 116693 |
| | | ARNO3, | | | | |
| | | CYTOHESIN-3, | | | | |
| | | GRP1, | | | | |
| | | KIAA4241, | | | | |
| | | MGC124579, | | | | |
| | | mKIAA4241, | | | | |
| | | Sec7, Sec7C | | | | |
| PTPRF | 200635_s_at, | AA591035, | protein tyrosine phosphatase, receptor type, F | 5792 | 19268 | 360406 |
| | 200637_s_at | FLJ43335, | | | | |
| | | FLJ45062, | | | | |
| | | FLJ45567, LAR, | | | | |
| | | Lar ptp2b, | | | | |
| | | LARFN5C, | | | | |
| | | LARS | | | | |
| PTPRM | | HR-PTPU, | protein tyrosine phosphatase, receptor type, M | 5797 | 19274 | 29616 |
| | 1555579_s_at | KIAA4044, | | | | |
| | | MGC90724, | | | | |
| | | mKIAA4044, | | | | |
| | | PTP-MU, | | | | |
| | | PTPRL1, R-PTP- | | | | |
| | | MU, RPTPM, | | | | |
| | | RPTPU | | | | |
| PVRL1 | 225211_at | AI835281, | poliovirus receptor-related 1 (herpesvirus entry mediator C; nectin) | 5818 | 58235 | 192183 |
| | | AW549174, | | | | |
| | | CD111, | | | | |
| | | CLPED1, ED4, | | | | |
| | | HIgR, HVEC, | | | | |
| | | MGC142031, | | | | |
| | | MGC16207, | | | | |
| | | NECTIN-1, | | | | |
| | | Nectin1 alpha, | | | | |
| | | Nectin1 delta, | | | | |
| | | OFC7, PRR, | | | | |
| | | PRR1, PVRR, | | | | |
| | | PVRR1, SK-12 | | | | |
| RAB40C | 227269_s_at | RAB40, RAR3, | RAB40C, member RAS | 57799 | 224624 | 359728 |
| | | RARL, RASL8C | oncogene family | | | |
| RASSF3 | 230466_s_at | AW212023, | Ras association | 283349 | 192678 | 362886 |
| | | AW322379, | (RalGDS/AF-6) domain family 3 | | | |
| | | MGC119194, | | | | |
| | | MGC119195, | | | | |
| | | MGC119197, | | | | |
| | | RASSF5 | | | | |
| RHOQ | 212120_at | ARHQ, | ras homolog gene family, member Q | 23433 | 104215 | 85428 |
| | | RASL7A, Rhot, | | | | |
| | | TC10, TC10 | | | | |
| | | BETA, TC10A | | | | |
| SAT1 | 203455_s_at, | AA617398, Ab2- | spermidine/spermine N1-acetyltransferase 1 | 6303 | 20229 | 302642 |
| | 210592_s_at, | 402, DC21, | | | | |
| | 213988_s_at, | KFSD, | | | | |
| | 230333_at | MGC72945, | | | | |
| | | SAT, | | | | |
| | | Spermidine/sper | | | | |
| | | mine N1-acetyl | | | | |
| | | transferase, | | | | |
| | | SSAT, SSAT-1 | | | | |
| SDCBP | 200958_s_at | MDA-9, ST1, | syndecan binding protein (syntenin) | 6386 | 53378 | 83841 |
| | | SYCL, | | | | |
| | | SYNTENIN, | | | | |
| | | Syntenin-1, | | | | |
| | | TACIP 18 | | | | |
| SEC61A1 | 217716_s_at, | AA408394, | Spec61 alpha I subunit (S. cerevisiae) | 29927 | 53421 | 80843 |
| | 222385_x_at | AA410007, | | | | |
| | | HSEC61, | | | | |
| | | rSEC61alpha p, | | | | |
| | | SEC61, Sec61 | | | | |
| | | alpha, SEC61 | | | | |
| | | ALPHA1, | | | | |
| | | SEC61A | | | | |
| SEMA3C | 236947_at | 1110036B02Rik, | sema domain, immunoglobulin domain | 10512 | 20348 | 296787 |
| | | SEMAE, | | | | |
| | | SEMAPHORIN | (Ig), short basic domain, secreted, (semaphorin) 3C | | | |
| | | E, SemE | | | | |
| SERGEF | 220482_s_at, | DELGEF, Gef, | secretion regulating guanine nucleotide exchange factor | 26297 | 27414 | 365243 |
| | 232983_s_at | Gnef, Gnefr, | | | | |
| | | MGC141208, | | | | |
| | | MGC141209, | | | | |
| | | RGD1563497 | | | | |
| SILV | 209848_s_at | D10H12S53E, | silver homolog (mouse) | 6490 | 20431 | 362818 |
| | | D12S53E, | | | | |
| | | D12S53Eh, | | | | |
| | | GP100, gp87, | | | | |
| | | ME20, PMEL17, | | | | |
| | | SI, SIL | | | | |
| SLC2A4RG | 227362 at | GEF, HDBP1, | SLC2A4 regulator | 56731 | | |
| | | Si-1-2, Si-1-2-19 | | | | |
| SLC7A1 | 212295_s_at | 4831426K01Rik, | solute carrier family 7 | 6541 | 11987 | 25648 |
| | | AI447493, | (cationic amino acid transporter, y+ system), member 1 | | | |
| | | ATRC1, CAT-1, | | | | |
| | | EcoR, ER, ERR, | | | | |
| | | HCAT1, mCAT- | | | | |
| | | 1, Rec-1, REC1L, | | | | |
| | | REV-1 | | | | |
| SRGAP2 | | 9930124L22Rik, | SLIT-ROBO Rho GTPase activating protein 2 | 23380 | 14270 | 360840 |
| | 1568957_x_at | AI448945, FBP2, | | | | |
| | | FNBP2, | | | | |
| | | KIAA0456, | | | | |
| | | RGD1566416, | | | | |
| | | srGAP3 | | | | |
| SSBP3 | 217991_x_at, | 2610421L12Rik, | single stranded DNA binding protein 3 | 23648 | 72475 | 84354 |
| | 223635_s_at | 2610200M23Rik, | | | | |
| | | 5730488C10Rik, | | | | |
| | | AI854733, | | | | |
| | | AW551939, | | | | |
| | | CSDP, | | | | |
| | | FLJ10355, | | | | |
| | | LAST, | | | | |
| | | MGC124589, | | | | |
| | | SSDP, SSDP1, | | | | |
| | | Ssdp3 | | | | |
| STAM | 203544_s_at | | signal transducing adaptor molecule (SH3 domain and ITAM motif) 1 | 8027 | 20844 | 498798 |
| (includes | | DKFZp686J2352, | | | | |
| EG:8027) | | RGD1564499, | | | | |
| | | Stam, STAM1 | | | | |
| SYNGR2 | 201079_at | CELLUGYRIN, | synaptogyrin 2 | 9144 | 20973 | 89915 |
| | | Clast2, | | | | |
| | | MGC102914 | | | | |
| TCF7L2 | 212759_s_at | mTcf-4B, mTcf- | transcription factor 7-like 2 (T-cell specific, HMG-box) | 6934 | 21416 | |
| (includes EG:6934) | | 4E, TCF-4, | | | | |
| | | TCF4B, TCF4E, | | | | |
| | | Tcf712 | | | | |
| TIMM17A | 215171_s_at | 17kDa, | translocase of inner mitochondrial membrane 17 homolog A (yeast) | 10440 | 21854 | 54311 |
| | | Mitochondrial | | | | |
| | | import inner | | | | |
| | | membrane | | | | |
| | | translocase, | | | | |
| | | Mitochondrial | | | | |
| | | protein import | | | | |
| | | protein 2, | | | | |
| | | mTim17a, | | | | |
| | | TIM17, TIM17A, | | | | |
| | | Timm17 | | | | |
| TP53 | 201746_at | bbl, bfy, bhy, | tumor protein p53 (Li-Fraumeni syndrome) | 7157 | 22059 | 24842 |
| | | N p53, | | | | |
| | | LFSI, | | | | |
| | | MGC112612, | | | | |
| | | P53, TRP53 | | | | |
| TPS3INP1 | 235602_at | 2700057G22Rik, | tumor protein p53 inducible nuclear protein 1 | 94241 | 60599 | 297822 |
| | | DKFZP434M131 | | | | |
| | | 7, FLJ22139, | | | | |
| | | p53DINP1, SIP, | | | | |
| | | SIP18, SIP27, | | | | |
| | | Stinp, Teap, | | | | |
| | | Thymus | | | | |
| | | Expressed Acidic | | | | |
| | | Protein, | | | | |
| | | TP53DINP1, | | | | |
| | | TP53DINP1alpha | | | | |
| | | TP53INP1A, | | | | |
| | | , TP53INP1B, | | | | |
| | | Trp53inp1 | | | | |
| TRIB2 | 202478_at | AW319517, | tribbles homolog 2 | 28951 | 217410 | 313974 |
| | | C5fw, GS3955, | (Drosophila) | | | |
| | | RGD1564451, | | | | |
| | | TRB-2 | | | | |
| TRPM1 | 237070_at | 4732499L03Rik, | transient receptor potential cation channel, subfamily M, member 1 | 4308 | 17364 | |
| (includes EG:4308) | | AI606771, | | | | |
| | | LTRPC1, | | | | |
| | | melastatin, | | | | |
| | | MLSN, MLSN1, | | | | |
| | | Trpm1 | | | | |
| TSPAN6 | 209108_at | 6720473L21Rik, | terraspanin 6 | 7105 | 56496 | 302313 |
| | | AI316786, | | | | |
| | | MGC117923, | | | | |
| | | T245, Tm4sf, | | | | |
| | | TM4SF6 | | | | |
| TSTA3 | 36936_at | AI256181, FX, | tissue specific transplantation antigen P35B | 7264 | 22122 | 300036 |
| | | FX protein, | | | | |
| | | MGC113801, | | | | |
| | | P35B, Tstap35b | | | | |
| TTC3 | 208073_x_at, | 2610202A04Rik, | tetratricopeptide repeat domain 3 | 7267 | 22129 | 360702 |
| | 210645_s_at | AA409221, | | | | |
| | | D16Ium21, | | | | |
| | | D16Ium21e, | | | | |
| | | DCRR1, | | | | |
| | | DKFZp686M015 | | | | |
| | | 0, KIAA4119, | | | | |
| | | mKIAA4119, | | | | |
| | | Mtprd, RNF105, | | | | |
| | | TPRD, TPRDIII | | | | |
| TUBB4 | 212664_at | AI325297, Beta | tubulin, beta 4 | 10382 | 22153 | 29213 |
| | | tubulin, BETA | | | | |
| | | TUBULIN 4 | | | | |
| | | ALPHA, Beta | | | | |
| | | tubulin class iv, | | | | |
| | | beta-5, Beta4 | | | | |
| | | Tubulin, | | | | |
| | | M(beta)4, Tubb, | | | | |
| | | TUBB5, | | | | |
| | | TUBULIN BETA | | | | |
| | | (5-BETA), | | | | |
| | | TUBULIN | | | | |
| | | BETA5 | | | | |
| TYR | 206630_at | albino, Dopa | tyrosinase | 7299 | 22173 | 308800 |
| | | oxidase, | (oculocutaneous albinism IA) | | | |
| | | Melanogenesis | | | | |
| | | Related | | | | |
| | | Tyrosinase, | | | | |
| | | OCA1A, OCAIA, | | | | |
| | | skc35, Tyr&It;c- | | | | |
| | | em>, | | | | |
| | | TYROSINASE | | | | |
| TYRP1 | 205694_at | b-PROTEIN, | tyrosinase-related protein 1 | 7306 | 22178 | 298182 |
| | | brown, CAS2, | | | | |
| | | CATB, GP75, | | | | |
| | | isa, | | | | |
| | | MELANOMA | | | | |
| | | ANTIGEN GP75, | | | | |
| | | TRP, TRP-1, | | | | |
| | | TYRP | | | | |
| VDR | 204255_s_at | NR1I1, VD3R, | vitamin D (1,25-dihydroxyvitamin D3) receptor | 7421 | 22337 | 24873 |
| | | VITAMIN D | | | | |
| | | RECEPTOR | | | | |
| VGLL4 | 214004_s_at | BC048841, | vestigial like 4 | 9686 | 232334 | 297523 |
| | | KIAA0121, | (Drosophila) | | | |
| | | MGC109514, | | | | |
| | | MGC54805, | | | | |
| | | VGL-4 | | | | |
| YIPF5 | 224949_at | 2610311I19Rik, | Yipl domain family, member 5 | 81555 | 67180 | 361315 |
| | | AA408236, Ac2- | | | | |
| | | 256, | | | | |
| | | DKFZp313L2216 | | | | |
| | | , FinGER5, | | | | |
| | | SB140, SMAP-5, | | | | |
| | | YIP1A | | | | |
| ZFHX1B | | 9130203F04Rik, | zinc finger homeobox 1b | 9839 | 24136 | 311071 |
| | 1557797_a_at | D130016B08Rik, | | | | |
| | , 203603_s_at | KIAA0569, | | | | |
| | | mKIAA0569, | | | | |
| | | SIP-1, | | | | |
| | | SMADIP1, | | | | |
| | | ZEB2, Zfx1b, | | | | |
| | | Zfxh1b | | | | |
| | 1558019_at | ---:Homo sapiens, | | | | |
| | | clone | | | | |
| | | IMAGE:4732650 | | | | |
| | | , mRNA | | | | |
| | 233551_at | LOC642776:hypo | | | | |
| | | thetical protein | | | | |
| | | LOC642776 | | | | |
| | 208646_at | RP14:ribosomal | | | | |
| | | protein S14 /// | | | | |
| | | similar to | | | | |
| | | ribosomal protein | | | | |
| | | S14 | | | | |
| | 208929_x_at | RPL13:ribosomal | | | | |
| | | protein L13 | | | | |
| | 214351_x_at | RPL13:ribosomal | | | | |
| | | protein L13 /// | | | | |
| | | similar to | | | | |
| | | ribosomal protein | | | | |
| | | L13 | | | | |
| | 200817_x_at | RPS10:ribosomal | | | | |
| | | protein S10 | | | | |
| | 213296_at | ---:Transcribed | | | | |
| | | locus | | | | |
| | 213692_s_at | ---:--- | | | | |
| | 227957_at | ---:--- | | | | |
| | 232462_s_at | FLJ23569:BC040 | | | | |
| | | 926 | | | | |
| | 227722_at | RPS23:ribosomal | | | | |
| | | protein S23 | | | | |
| | 217466_x_at | RPS2:ribosomal | | | | |
| | | protein S2 /// | | | | |
| | | similar to | | | | |
| | | ribosomal protein | | | | |
| | | S2 | | | | |
| | 235534_at | ---:Homo sapiens, | | | | |
| | | clone | | | | |
| | | IMAGE:5723825 | | | | |
| | | , mRNA | | | | |
| | 230741_at | ---: Full length | | | | |
| | | insert cDNA | | | | |
| | | clone YX74D05 | | | | |
| | 229067_at | LOC653464:Simi | | | | |
| | | lar to SLIT- | | | | |
| | | ROBO Rho | | | | |
| | | GTPase- | | | | |
| | | activating protein | | | | |
| | | 2 (srGAP2) | | | | |
| | | (Formin0binding protein 2) | | | | |
| | | | | | | |

**Table 2**

| name | matched term |
|---|---|
| ANKRD44 | 228471 at |
| ARHGEF19 | 226857 at |
| ATPBD4 | 238662_at |
| BARX2 | 210419_at |
| BDNF | 206382 s at |
| BLOC1S1 | 202592_at |
| C16ORF48 | 223407 at |
| C60RF218 | 244829_at |
| C80RF13 | 233641 s at |
| CCHCR1 | 37425_g_at |
| CIRBP | 230142_s_at |
| CLSTN2 | 219414 at |
| COL7A1 | 217312 s at |
| DACH1 | 205472 s at, 228915 at |
| DCT | 205337 at, 205338_s_at |
| DOCK10 | 219279_at |
| DRAP1 | 15S6181_at |
| EDNRB | 204271_s_at, 206701 x at |
| EFNA4 | 205107_s_at |
| EHD2 | 45297_at |
| ETS1 | 224833_at |
| FAM33A | 225684_at |
| FGFR1 | 210973_s_at, 211535 s at |
| FO7CO1A | 202723 s at |
| GGA3 | 209411 s at |
| GPR161 | 214104 at |
| HIST2H2AA3 | 214290 s at |
| ID1 | 208937_s_at |
| KDELR1 | 200922_at |
| KIAA0738 | 210529 s at |
| KIT | 205051_s at |
| LGR4 | 230674_at |
| LHX2 (includes EG:9355) | 211219 s at |
| LMO4 | 209204_at |
| LOC254100 | 1557131_at |
| LRIG1 | 238339 x at |
| MED28 | 222635_s_at |
| MKL1 | 215292_s_at |
| MLANA | 206426_at, 206427 s at |
| MLPH | 218211_s_at |
| MYEF2 | 222771_s_at, 232676 x at |
| MYOSA | 227761_at |
| NBL1 | 37005_at |
| OSTM1 | 218196_at |
| PDK3 | 221957 at |
| PKD1 | 241090_at |
| PLEKHA5 | 220952_s_at |
| PLP1 | 210198_s_at |
| PLXNC1 | 213241 at |
| PRKCSH | 200707 at |
| PRKD3 | 222565 s at |
| PRMT1 | 206445 s at |
| PSCD3 | 225147_at |
| PTPRF | 200637_s_at |
| PTPRM | 1555579 s at |
| RAB40C | 227269_s_at |
| RASSF3 | 230466 s at |
| RHOQ | 212120_at |
| RPL13 | 214351_x_at |
| RPS23 | 227722 at |
| SAT1 | 203455_s_at, 213988_s_at, 230333_at |
| SDCBP | 200958_s_at |
| SEC61A1 | 222385_x_at |
| SEMA3C | 236947_at |
| SERGEF | 232983_s_at |
| SILV | 209848_s_at |
| SLC2A4RG | 227362 at |
| SLC7A1 | 212295_s_at |
| SSBP3 | 217991_x_at, 223635_s_at |
| STAM (includes EG:8027) | 203544_s_at |
| SYNGR2 | 201079 at |
| TCF7L2 (includes EG:6934) | 212759 s at |
| TIMM17A | 215171_s_at |
| TRIB2 | 202478_at |
| TRIM1 (includes EG:4308) | 237070 at |
| TSPAN6 | 209108_at |
| TTC3 | 208073_x_at, 210645_s_at |
| TUBB4 | 212664_at |
| TYR | 206630_at |
| VDR | 204255 s at |
| YIPF5 | 224949 at |
| ZFHX1B | 1557797 a at, 203603_s_at |
| | 229067 at |
| | 213692 s at |
| | 227957 at |
| | 213296_at |
| | 235534_at |
| | 233551 at |
| | 1558019 at |

**Table 3**

| matched term | description |
|---|---|
| 208073 x at | TTC3 :tetratricopeptide repeat domain 3 |
| 210545_s_at | TTC3:tetratricopeptide repeat domain 3 |
| 206630 at | TYR:tyrosinase (oculocutaneous albinism IA) |
| 203544 s at | STAM:signal transducing adaptor molecule (SH3 domain and ITAM motif) 1 |
| 230741_at | ---:Full length insert cDNA clone YX74D05 |

**Table 4**

| matched term | description |
|---|---|
| 205694 at | TYRP1:tyrosinase-related protein 1 |
| 206427_s_at | MLANA:melan-A |
| 206140_at | LHX2:LIM homeobox 2 |
| 206630_at | TYR:tyrosinase (oculocutaneous albinism IA) |
| 203921_at | CHST2:carbohydrate (N-acetylglucosamine-6-O) sulfotransferase 2 |
| 205337_at | DCT:dopachrome tautomerase (dopachrome delta-isomerase, tyrosine-related protein 2) |
| 228245_s_at | OVOS2:ovostatin 2 /// similar to cDNA sequence BC048546 |
| 205338_s_at | DCT:dopachrome tautomerase (dopachrome delta-isomerase, tyrosine-related protein 2) |
| 1557797 a at | ZFHX1B:Zinc finger homeobox 1b |
| 204271_s_at | EDNRB:endothelin receptor type B |
| 237070_at | TRPM1:transient receptor potential cation channel, subfamily M, member 1 |
| 200716_x_at | RPL13A:ribosomal protein L13a |
| 1555579_s_at | PTPRM:protein tyrosine phosphatase, receptor type, M |
| 205051_s_at | KIT-v-kit Hardy-Zuckerman 4 feline sarcoma viral oncogene homolog |
| 200665_s_at | SPARC:secreted protein, acidic, cysteine-rich (osteonectin) /// secreted protein, acidic, cysteine-rich (osteonectin) |
| 205174_s_at | QPCT:glutaminyl-peptide cyclotransferase (glutaminyl cyclase) |
| 200725_x_at | RP10:ribosomal protein L10 |
| 232602_at | WFDC3:WAP four-disulfide core domain 3 |
| 202478 at | TRIB2:tribbles homolog 2 (Drosophila) |
| 209230_s_at | P8:p8 protein (candidate of metastasis 1) |
| 232676_x_at | MYEF2:myelin expression factor 2 |
| 222565_s_at | PRKD3:protein kinase D3 |
| 212295_s_at | SLC7A1: solute carrier family 7 (cationic amino acid transporter, y+ system), member 1 |
| 212594 at | PDCD4:programmed cell death 4 (neoplastic transformation inhibitor) |
| 218211_s_at | MLPH:melanophilin |
| 206426 at | MLANA:melan-A |
| 207065 at | K6HF:cytokeratin type II |
| 202500_at | DNAJB2:DnaJ (Hsp40) homolog, subfamily H, member 2 |
| 203706_s_at | FZD7:frizzled homolog 7 (Drosophila) |
| 209969_s_at | STAT1:signal transducer and activator of transcription 1, 91kDa |

**Table 5**

| matched term | description |
|---|---|
| 205694_at | tyrosinase-related protein 1 |
| 206140_at | LIM homeobox 2 |
| 206427_s_at | melan-A |
| 203455_s_at | spermidine/spermine N1-acetyltransferase |
| 206453_s_at | NDRG family member 2 |
| 203921_at | carbohydrate (N-acetylglueosamine-6-O) sulfotransferase 2 |
| 200958_s_at | syndecan binding protein (syntenin) |
| 209283_at | crystallin, alpha B |
| 204271_s_at | endothelin receptor type B |
| 208073_x_at | tetratricopeptide repeat domain 3 |
| 232602_at | WAP four-disulfide core domain 3 |
| 202435_s_at | cytochrome P450, family 1, subfamily B, polypeptide I |
| 209230_s_at | p8 protein (candidate of metastasis 1) |
| 208966_x_at | interferon, gamma-inducible protein 16 |
| 205337 at | dopachrome tautomerase (dopachrome delta-isomerase, tyrosine-related protein 2) |
| 202088 at | solute carrier family 39 (zinc transporter), member 6 |
| 211538_s_at | heat shock 70kDa protein 2 |
| 201556_s_at | vesicle-associated membrane protein 2 (synaptobrevin 2) |
| 241455_at | Similar to AI661453 protein |
| 237070 at | transient receptor potential cation channel, subfamily M, member 1 |

**Table 6**

| matched term | description |
|---|---|
| 1555505_a_at | tyrosinase (oculocutaneous albinism 1A) |
| 1204271_s_at | endothelin receptor type B |
| 208073 x at | tetratricopeptide repeat domain 3 |
| 200958_s_at | syndecan binding protein (syntenin) |
| 205051_s_at | v-kit Hardy-Zuckerman 4 feline sarcoma viral oncogene homolog |
| 201245_s_at | OTU domain, ubiquitin aldehyde binding 1 |
| 201603 at | protein phosphatase 1, regulatory (inhibitor) subunit 12A |
| 201605_x_at | calponin 2 |
| 201908_at | dishevelled, dsh homolog 3 (Drosophila) |
| 202478_at | tribbles homolog 2 (Drosophila) |
| 1557292 a at | mucolipin 3 |
| 200601_at | actinin, alpha 4 |
| 200819_s_at | ribosomal protein S15 |
| 209953_s_at | CDC37 cell division cycle 37 homolog (*S. cerevisiae*) |
| 213146_at | jumonji domain containing 3 |
| 222670_s_at | v-maf musculoaponeurotic fibrosarcoma oncogene homolog B (avian) |
| 224991_at | c-Maf-inducing protein |
| 226988_s_at | myosin, heavy polypeptide 14 |
| 244829 at | Hypothetical protein MGC40222 |

Although the invention has been described with reference to the above examples, it will be understood that modifications and variations are encompassed within the spirit and scope of the invention. Accordingly, the invention is limited only by the following claims.
Aspects and features of the present disclosure are set out in the following numbered clauses which contain the subject matter of the claims of the parent application as filed.
1. A method for characterizing a skin lesion in a subject comprising analyzing a nucleic acid molecule from one or more genes listed in any of Tables 1-6, or any combination thereof, in a sample of the skin lesion, thereby characterizing a skin lesion of the subject.
2. The method of clause 1 , wherein the nucleic acid molecule is RNA.
3. The method of clause 1 , wherein analyzing the nucleic acid molecule comprises detecting one or more mutations in the nucleic acid sequence of the nucleic acid molecule.
4. The method of clause 3, wherein the one or more mutations are selected from the group consisting of a substitution, a deletion, and an insertion.
5. The method of clause 1, further comprising amplifying the nucleic acid molecule obtained from the sample prior to analyzing.
6. The method of clause 1, wherein the sample is obtained by applying an adhesive tape to a target area of skin in a manner sufficient to isolate the sample adhering to the adhesive tape.
7. The method of clause 1, further comprising using the characterizing to determine a treatment regimen.
8. The method of clause 2, wherein the isolated nucleic acid molecule or an amplification product thereof, is applied to a microarray.
9. The method of clause 8, wherein an expression profile is detected using a microarray.
10. The method of clause 1, wherein the sample is obtained from a biopsy taken at the site of the skin lesion or surrounding margin.
11. The method of clause 6, wherein the tape comprises a rubber adhesive on a polyurethane film.
12. The method of clause 6, wherein about one to ten adhesive tapes or one to ten applications of a tape are applied and removed from the skin.
13. The method of clause 6, wherein about one to eight adhesive tapes or one to eight applications of a tape are applied and removed from the skin.
14. The method of clause 6, wherein about one to five adhesive tapes or one to five applications of a tape are applied and removed from the skin.
15. The method of clause 6, wherein the method further comprises taking a biopsy of the target area of the skin.
16. The method of clause 2, wherein the analyzing is performed in situ.
17. The method of clause 1 , wherein the nucleic acid molecule is from one or more genes listed in Table 6.
18. A method of distinguishing melanoma from dysplastic nevi or normal pigmented skin in a subject comprising analyzing a nucleic acid molecule from one or more genes listed in any of Tables 1-6, or any combination thereof, in a sample from the subject, thereby distinguishing melanoma from dysplastic nevi or normal pigmented skin in a subject.
19. The method of clause 18, wherein the nucleic acid molecule is RNA.
20. The method of clause 18, wherein analyzing the nucleic acid molecule comprises detecting one or more mutations in the nucleic acid sequence of the nucleic acid molecule.
21. The method of clause 20, wherein the one or more mutations are selected from the group consisting of a substitution, a deletion, and an insertion.
22. The method of clause 18, further comprising amplifying the nucleic acid molecule obtained from the sample prior to analyzing.
23. The method of clause 18, wherein the sample is obtained by applying an adhesive tape to a target area of skin in a manner sufficient to isolate the sample adhering to the adhesive tape, wherein the sample comprises nucleic acid molecules.
24. The method of clause 19, wherein the isolated nucleic acid molecule or an amplification product thereof, is applied to a microarray.
25. The method of clause 22, wherein an expression profile is detected using a microarray.
26. The method of clause 18, wherein the sample is obtained from a biopsy taken from a target area of skin from the subject.
27. The method of clause 23, wherein the tape comprises a rubber adhesive on a polyurethane film.
28. The method of clause 23, wherein about one to ten adhesive tapes or one to ten applications of a tape are applied and removed from the skin.
29. The method of clause 23, wherein about one to eight adhesive tapes or one to eight applications of a tape are applied and removed from the skin,
30. The method of clause 23, wherein about one to five adhesive tapes or one to five applications of a tape are applied and removed from the skin.
31. The method of clause 23, wherein the method further comprises taking a biopsy of the target area of the skin.
32. The method of clause 19, wherein the analyzing is performed in situ.
33. A method for diagnosing melanoma in a subject comprising detecting an altered level of a target protein in a sample from the subject, as compared to the level of the target protein in a corresponding sample from a subject that does not have melanoma, wherein the protein is an expression product of a gene listed in any of Tables 1-6, or any combination thereof, thereby diagnosing melanoma in the subject.
34. The method of clause 33, wherein the sample is obtained by applying an adhesive tape to a target area of skin in a manner sufficient to isolate the sample adhering to the adhesive tape, wherein the sample comprises cells, and further comprising lysing the cells to extract the target protein.
35. The method of clause 34, wherein the tape comprises a rubber adhesive on a polyurethane film.
36. The method of clause 34, wherein between one and ten adhesive tapes are applied to the skin and removed from the skin.
37. The method of clause 34, wherein about one to eight adhesive tapes are applied and removed from the skin.
38. The method of clause 34, wherein about one to five adhesive tapes are applied and removed from the skin.
39. The method of clause 34, wherein the method further comprises taking a biopsy of the target area of the skin.
40. The method of clause 39, wherein protein is extracted from the biopsy sample, and the level of protein in the biopsy and the level of protein in the tape sample are analyzed.
41. The method of clause 33 , wherein the sample is obtained by from a biopsy of a target area of skin.
42. The method of clause 33, further comprising obtaining a sample from uninvolved tissue of the subject.
43. The method of clause 42, wherein the sample from uninvolved tissue is obtained by taking a biopsy of the uninvolved skin.
44. The method of clause 42, wherein the sample from uninvolved tissue is obtained by: a) applying an adhesive tape to skin of the subject in a manner sufficient to isolate a skin sample adhering to the adhesive tape, wherein the skin sample comprises cells from the stratum corneum and wherein the skin is unaffected by a disease to be tested; b) lysing the cells to extract a protein; and c) quantitating the extracted protein.
45. The method of clause 42, wherein the uninvolved skin is from the mastoid process or the upper back.
46. The method of clause 33, wherein the protein is an expression product of a gene listed in Table 6.
47. A method for diagnosing melanoma in a subject, comprising: a) providing a gene expression profile of a target area suspected of being melanoma on the skin of the subject, wherein the target area of the skin simultaneously expresses a plurality of genes at the protein level that are markers for melanoma; and b) comparing the subject's gene expression profile to a reference gene expression profile obtained from a corresponding normal skin sample, wherein the reference gene expression profile comprises an expression value of a target gene selected from the group consisting of human niRNA for tyrosinase- related protein 1, LIM homeobox 2, melan-A, spermidine/spermine N1- acetyltransferase, NDRG family member 2, carbohydrate (N- acetylglucosamine-6-O) sulfotransferase 2, syndecan binding protein (syntenin), crystallin, alpha B, endothelin receptor type B, tetratricopeptide repeat domain 3, WAP four-disulfide core domain 3, cytochrome P450 (family 1, subfamily B, polypeptide 1), p8 protein (candidate of metastasis 1), interferon gamma-inducible protein 16, dopachrome tautomerase (dopachrome delta-isomerase, tyrosine-related protein 2), solute carrier family 39 (zinc transporter), member 6, heat shock 7OkDa protein 2, vesicle-associated membrane protein 2 (synaptobrevin 2), Similar to AI661453 protein, and transient receptor potential cation channel (subfamily M, member 1), tyrosinase (oculocutaneous albinism IA), endothelin receptor type B, tetratricopeptide repeat domain 3, syndecan binding protein (syntenin), v-kit Hardy-Zuckerman 4 feline sarcoma viral oncogene homolog, OTU domain, ubiquitin aldehyde binding 1 , protein phosphatase 1 regulatory (inhibitor) subunit 12 A, calponin 2, dishevelled dsh homolog 3 (Drosophila), tribbles homolog 2 (Drosophila), mucolipin 3, actinin alpha 4, ribosomal protein S 15, CDC37 cell division cycle 37 homolog (S. cerevisiae), jumo[eta]ji domain containing 3, v-maf musculoaponeurotic fibrosarcoma oncogene homolog B (avian), c-Maf-inducing protein, myosin heavy polypeptide 14, Hypothetical protein MGC40222, or any combination thereof.
48. The method of clause 47, wherein the reference gene expression profile further comprises an expression value of any target gene listed in Tables 1-6.
49. The method of clause 47 or 48, wherein the reference gene expression profile is contained within a database.
50. The method of clause 47, wherein said comparing is carried out using a computer algorithm.
51. A kit for characterizing a skin lesion in a subject comprising a skin sample collection device and one or more probes or primers that selectively bind to one or more nucleic acid molecules in any of Tables 1-6, or to a nucleic acid or protein expression product of a nucleic acid molecule in any of Tables 1-6.
52. The kit of clause 51 , wherein the kit provides a probe which binds to a portion of a nucleic acid molecule in any of Tables 1-6.
53. The kit of clause 51 , wherein the kit provides one or more primer pairs comprising a forward primer that selectively binds upstream of a gene on one strand and a reverse primer that selectively binds upstream of a gene on a complementary strand, wherein the gene is listed in any of Tables 1-6.
54. The kit of clause 51, wherein the skin sample collection device is a biopsy needle.
55. The kit of clause 51 , wherein the skin sample collection device is an adhesive tape.
56. The kit of clause 55, wherein the adhesive tape comprises a rubber adhesive on a polyurethane film.
57. The kit of clause 51, further comprising a microarray containing at least a fragment of a gene or a nucleic acid or protein product of a gene identified in any of Tables 1-6 or any combination thereof.
58. A kit for characterizing a skin lesion in a subject comprising an applicator and one or more probes or primers that selectively bind to one or more of nucleic acid molecules in any of Tables 1-6, or to a nucleic acid or protein expression product of a nucleic acid molecule in any of Tables 1-6.
59. The kit of clause 58, wherein the probes are detectably labeled.
60. The method of any one of clauses 1, 18, 33, or 47, wherein the subject is human.
61. The method of any one of clauses 1 , 18, 33, or 47, wherein the sample is an epidermal sample.

## Claims

1. A non-invasive method of distinguishing melanoma from non-melanoma in a human subject comprising analyzing a nucleic acid molecule from a gene, in a sample obtained from the subject by a non-invasive technique, wherein the gene is one or more of tetratricopeptide repeat domain 3, actinin alpha 4, Hypothetical protein MGC40222, endothelin receptor type B or OTU domain ubiquitin aldehyde binding 1, and wherein a difference in expression level of the gene as compared to expression in non-melanoma is indicative of melanoma, thereby distinguishing melanoma from non-melanoma in the subj ect.

2. The method of claim 1, wherein the nucleic acid molecule is RNA.

3. The method of claim 1, further comprising amplifying the nucleic acid molecule obtained from the sample prior to analyzing.

4. The method of claim 1, wherein the sample is obtained by applying an adhesive tape to a target area of skin in a manner sufficient to isolate the sample adhering to the adhesive tape.

5. The method of claim 1, further comprising using the characterizing to determine a treatment regimen.

6. The method of claim 2, wherein the isolated nucleic acid molecule or an amplification product thereof, is applied to a microarray.

7. The method of claim 6, wherein an expression profile is detected using a microarray.

8. The method of claim 4, wherein the tape comprises a rubber adhesive on a polyurethane film.

9. The method of claim 4, wherein about one to ten adhesive tapes or one to ten applications of a tape are applied and removed from the skin.

10. The method of claim 4, wherein about one to eight adhesive tapes or one to eight applications of a tape are applied and removed from the skin.

11. The method of claim 4, wherein about one to five adhesive tapes or one to five applications of a tape are applied and removed from the skin.

12. The method of claim 2, wherein the analyzing is performed *in situ.*

13. The method of claim 1, further comprising analyzing a nucleic acid molecule from one or more genes listed in Table 6.
